# EUROPEAN PATENT APPLICATION

(11) **EP 4 393 907 A1**
(43) Date of publication of application: **03.07.2024**
(21) Application number: 22860622.4
(22) Date of filing: 26.08.2022
(51) Int. Cl.: C07D 223/14, C07D 225/06, C07D 495/04, C07D 407/04, C07D 513/04, C07D 403/04, A61K 31/55, A61K 31/395, A61K 31/403, A61P 3/12, A61P 25/00, A61P 25/28, A61P 25/08, A61P 25/04, A61P 25/06

(54) **POTASSIUM CHANNEL MODULATOR, COMPOSITION AND APPLICATION**

(30) Priority: 26.08.2021 CN 202110988126; 07.04.2022 CN 202210361600
(71) Applicant: Neushen Therapeutics (Shanghai) Co., Ltd, Shanghai 201210 (CN)
(72) Inventor: HU, Miao, Hangzhou, Zhejiang 310018 (CN); WU, Yizhe, Hangzhou, Zhejiang 310018 (CN); LIU, Xingguo, Hangzhou, Zhejiang 310018 (CN); ZHOU, Xinglu, Hangzhou, Zhejiang 310018 (CN)
(74) Representative: Haseltine Lake Kempner LLP
(86) International application number: PCT/CN2022/115057
(87) International publication number: WO 2023/025276

(57) **Abstract**

Disclosed in the present invention is a compound having a structure shown in general formula I, or a stereoisomer thereof, a pharmaceutically acceptable salt thereof, or a solvate thereof. Further disclosed in the present invention are a composition comprising the compound, a preparation and an application. The compound of the present invention has a significant activation effect on KCNQ2/3 potassium ions, and can be used for treating diseases related to potassium channel ion flow, especially for treating central nervous system diseases.

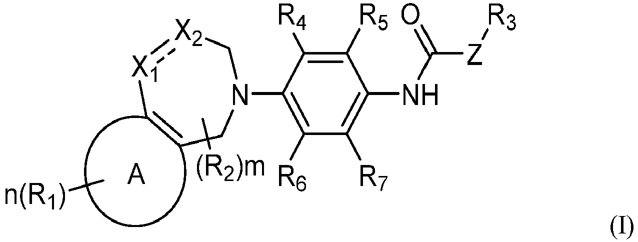

## Description

### TECHNICAL FIELD

The present disclosure belongs to the technical field of drug synthesis and design, and in particular relates to a class of compounds regulating potassium channels, a stereoisomer thereof, a pharmaceutically acceptable salt thereof, a pharmaceutical composition thereof, and a use thereof. The compound has the function of regulating potassium channels and can be used for treating diseases or disorders benefiting from KCNQ channel openers.

### BACKGROUND

The treatment of central nervous system diseases has always been a difficult problem in the medical field. Traditional drugs cannot prevent and treat acute and chronic central nervous system injuries, Alzheimer's disease, Parkinson's disease, epilepsy, and other central nervous system diseases.

The chemical environment of cells is closely related to the ion channels on the cell membrane. Ion channels are the key to regulating the excitability of neurons. Therefore, ion channels have become the most direct targets for the treatment of central nervous system diseases. Approximately more than 10% of potassium ion channel subtypes are known to be associated with central nervous system diseases in humans in many different ways, for example, from direct control of neuronal excitability and homeostasis of the ionic environment to indirect effects through metabolism.

At present, five types of KCNQ1 to 5 have been found. KCNQ potassium ion channel is an important branch of the potassium ion channel superfamily, and the gene mutation thereof is related to many genetic diseases. Among them, KCNQ1 (KvLQT) is mainly distributed in the myocardium, and 50% of hereditary LQT syndromes are related to KCNQ1 mutations. KCNQ2 and KCNQ3 are the molecular basis of M-type potassium ion channels in nerve cells. Benign familial neonatal convulsion (BFNC) is related to the downregulation of M currents caused by KCNQ2 and KCNQ3 gene mutations. KCNQ4 is mostly expressed in nerve conduction pathways, nerve nuclei, and inner ear hair cells related to hearing. Dominant nonsyndromic hearing loss (DFNA) is related to the mutation of KCNQ4 gene. KCNQ5 is mostly expressed in muscle tissue.

KCNQ potassium channel opener has become a new direction of antiepileptic drug research. Retigabine was approved by the US FDA in June 2011 for the treatment of partial seizures in refractory epilepsy. Its excellent performance *in vivo* and *in vitro* and its successful marketing prove that the KCNQ potassium channel is of great significance as a drug target. Moreover, *in vivo* and *in vitro* studies have shown that Retigabine also has potential therapeutic effects on anxiety, stroke, neurodegenerative diseases, and pain. At the same time, due to the wide range of physiological functions of the KCNQ potassium channel, the opener thereof also has very broad application prospects in the treatment of many diseases.

### CONTENT OF THE PRESENT INVENTION

The present disclosure is to provide a novel compound, a pharmaceutically acceptable salt thereof, or a solvate thereof with the function of regulating potassium channels.

The present disclosure also provides a pharmaceutical composition comprising the compound, a stereoisomer thereof, a stereoisomer mixture thereof, or a pharmaceutically acceptable salt thereof.

The present disclosure also provides a use of the compound, the stereoisomer thereof, the stereoisomer mixture thereof, or the pharmaceutically acceptable salt thereof in the manufacture of a medicament for treating the disease, disorder, or condition benefiting from a KCNQ channel opener.

The compounds of the present disclosure are effective in treating and preventing diseases and conditions affected by the activity of potassium ion channels and can be used to treat various diseases and disorders.

To achieve the above object, the present disclosure provides a compound of formula I, 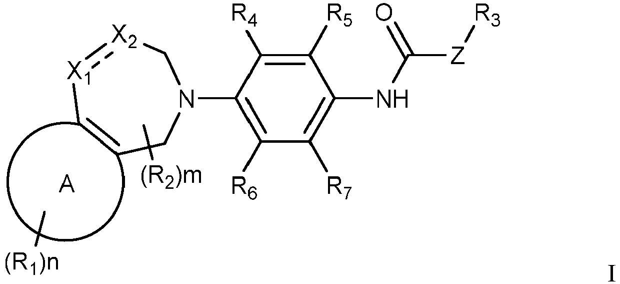
or a stereoisomer thereof, or a pharmaceutically acceptable salt thereof;
wherein
ring A is a benzene ring or a 5- to 8-membered heterocycle containing 1 to 2 heteroatoms selected from N, O, or S;
R₁ is selected from hydrogen, halogen, C₁-C₈ alkyl, C₁-C₈ alkoxy, C₁-C₈ alkenyl, C₁-C₈ alkenoxy, spiroalkyl, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₁-C₈ alkenoxy, halo C₃-C₈ cycloalkyl, halo C₃-C₈ cycloalkoxy, halo C₂-C₈ heterocycloalkyl, halo C₂-C₈ heterocycloalkoxy, and the alkyl, alkoxy, alkenyl, alkenoxy, cycloalkyl, cycloalkoxy, heterocycloalkyl, heterocycloalkoxy, alkylthio, alkanoyl, alkylsulfonyl, aminosulfonyl are unsubstituted or substituted by R₈;
each R₂ is independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
or two R₂ groups are attached to the same atom, and the two R₂ groups are different or the same, and the two R₂ groups, together with the atom to which they are attached, form a 3- to 6-membered ring or a 3- to 6-membered heterocycle;
R₄, R₅, R₆, R₇, R₈ are each independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy, spiroalkyl;
n is selected from 0, 1, 2;
m is selected from 0, 1, 2, 3, 4, 5;
X₁ and X₂ are each independently selected from -CRaRb, -NRa, -O-, -C(O)-, -S-, - S(O)-, -S(O)₂-;
Ra and Rb are each independently selected from hydrogen, halogen, hydroxyl, amino, C₁-C₈ alkyl, C₁-C₈ alkoxy, C₁-C₈ alkylamino, C₃-C₈ cycloalkyl, halo C₁-C₈ alkyl, halo C₁-C₈ alkoxy, halo C₃-C₈ cycloalkyl;
--- indicates the presence or absence of a chemical bond;
Z is selected from O or (CH₂)ₚ, and p is an integer of 1 to 6;
R₃ is C₁-C₈ alkyl, C₃-C₈ cycloalkyl, C₃-C₈ cycloalkenyl, C₂-C₈ alkenyl, or C₂-C₈ alkynyl, wherein the C₁-C₈ alkyl, C₃-C₈ cycloalkyl, C₃-C₈ cycloalkenyl, C₂-C₈ alkenyl, or C₂-C₈ alkynyl is unsubstituted or substituted by one or more than one substituent selected from halogen, nitro, cyano, amino, or hydroxyl.

Preferably, the compound of the present disclosure further has a structure shown in general formula I:
or a stereoisomer thereof, or a pharmaceutically acceptable salt thereof;
wherein
ring A is a benzene ring or a 5- to 8-membered heterocycle containing 1 to 2 heteroatoms selected from N, O, or S;
each R₁ is independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
each R₂ is independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
R₄, R₅, R₆, R₇ are each independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
n is selected from 0, 1, 2;
m is selected from 0, 1, 2, 3, 4, 5;
X₁ and X₂ are each independently selected from -CRaRb, -NRa, -O-, -C(O)-, -S-, - S(O)-, -S(O)₂-;
Ra and Rb are each independently selected from hydrogen, halogen, hydroxyl, amino, C₁-C₈ alkyl, C₁-C₈ alkoxy, C₁-C₈ alkylamino, C₃-C₈ cycloalkyl, halo C₁-C₈ alkyl, halo C₁-C₈ alkoxy, halo C₃-C₈ cycloalkyl;
- - - indicates the presence or absence of a chemical bond;
Z is selected from O or (CH₂)ₚ, and p is an integer of 1 to 6;
R₃ is C₁-C₈ alkyl, C₃-C₈ cycloalkyl, C₃-C₈ cycloalkenyl, C₂-C₈ alkenyl, or C₂-C₈ alkynyl, wherein the C₁-C₈ alkyl, C₃-C₈ cycloalkyl, C₃-C₈ cycloalkenyl, C₂-C₈ alkenyl, or C₂-C₈ alkynyl is unsubstituted or substituted by one or more than one substituent selected from halogen, nitro, cyano, amino, or hydroxyl;
when 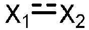 is represented as X₁=X₂ , X₁, X₂ are each independently selected from -CRa-, -N-;
when 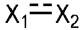 is represented as X₁-X₂, and when none of R₄, R₅, R₆, R₇ is selected from chlorine, and when X₁ is CH or O, R₂ is H, and when ring A is a benzene ring, R₁ is selected from C₁-C₈ alkoxy, C₃-C₈ cycloalkoxy, C₂-C₈ heterocycloalkoxy, C₁-C₈ alkylthio, C₁-C₈ alkanoyl, C₁-C₈ alkylsulfonyl, aminosulfonyl, halo C₁-C₈ alkoxy, halo C₃-C₈ cycloalkoxy, halo C₂-C₈ heterocycloalkoxy;
when 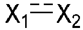 is represented as X₁-X₂, and when none of R₄, R₅, R₆, R₇ is selected from chlorine, and when ring A is a thiophene ring, m is selected from 2, 3, 4, 5, and R₂ is not selected from hydrogen.

In the present disclosure, when n and m are greater than 1, n and m are multi-substituted; at this time, multiple substituents can be substituted on one carbon atom or on multiple atoms of the corresponding ring structure; and when multi-substituted, multiple substituents can be the same or different. When two substituents are attached to the same carbon atom, and the two substituents, together with the carbon atom to which they are attached, can form a C₃-C₆ cycloalkyl ring.

Preferably, ring A is a benzene ring or a 5-membered heterocycle containing 1 to 2 heteroatoms selected from N, O, or S.

Preferably, R₁ is selected from hydrogen, C₁-C₅ alkoxy, C₁-C₅ alkenoxy, halo C₁-C₅ alkoxy, C₃-C₆ cycloalkyl, C₃-C₆ cycloalkoxy, C₃-C₄ heterocycloalkyl, C₃-C₄ heterocycloalkoxy, C₁-C₄ alkylthio, halogen, C₁-C₄ alkylsulfonyl, and R₁ is unsubstituted or substituted by one or more than one substituent selected from halogen, C₁-C₃ alkyl, halo C₁-C₃ alkyl, C₁-C₃ alkoxy, halo C₁-C₃ alkoxy, spiroalkyl. Preferably, R₁ is selected from H, C₁-C₄ alkoxy, halo C₁-C₄ alkoxy, C₃-C₆ cycloalkyl, C₃-C₄ heterocycloalkyl, C₁-C₄ alkylthio, halogen, C₁-C₄ alkylsulfonyl. More preferably, R₁ is H, F, Cl, methoxy, ethoxy, propoxy, isopropoxy, butoxy, isobutoxy, sec-butoxy, difluoromethoxy, trifluoromethxoy, trifluoroethoxy, trifluoropropoxy, trifluoroisopropoxy, hexafluoroisopropoxy, trifluorobutoxy, cyclopropoxy, cyclobutoxy, cyclopentyloxy, cyclohexacyloxy, oxetanyloxy, methylthio, methylsulfonyl,

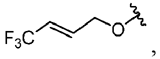

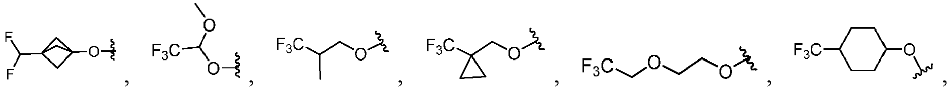

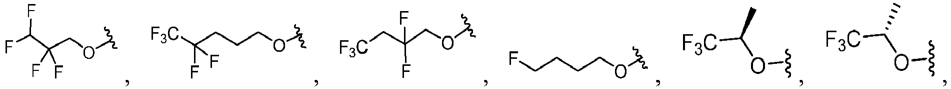

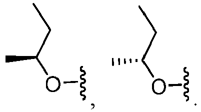

Preferably, each R₂ is independently selected from hydrogen, halogen, C₁-C₃ alkyl. More preferably, R₂ is H or methyl. Even more preferably, R₂ is H or a methyl substituent on X₁. Even more preferably, R₂ is H, or one or two methyl substituents on X₁, or a cycloalkyl group formed by two methyl groups together with X₁.

Preferably, R₃ is C₁-C₅ alkyl. More preferably, R₃ is tert-butyl.

Preferably, R₄, R₅, R₆, R₇ are each independently H, methyl, halogen (more preferably Cl). More preferably, R₄, R₆ are H or Cl; R₅, R₇ are methyl.

Preferably, Z is (CH₂)ₚ, more preferably, Z is -CH₂-.

More preferably, the compound of the present disclosure has a structure shown in formula IIa, IIb, or IIc: 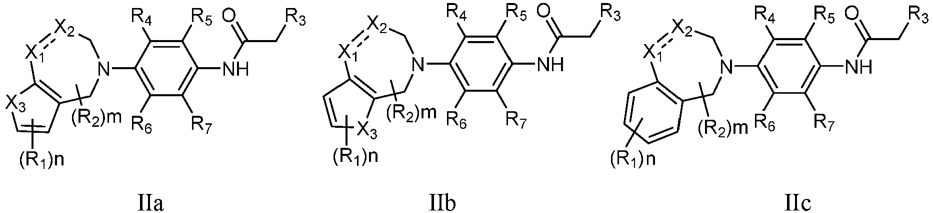
or a stereoisomer thereof, or a pharmaceutically acceptable salt thereof;
the bond between X₁ and X₂ is a double bond or a single bond in the general formulas.

Preferably, X₃ is N, O, or S.

More preferably, the compound of the present disclosure has a structure shown in general formula III: 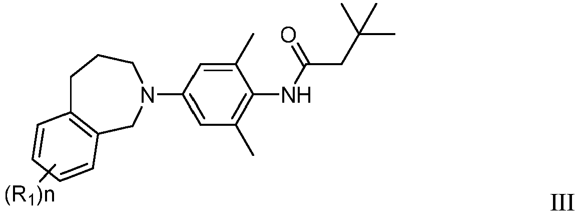
or a stereoisomer thereof, or a pharmaceutically acceptable salt thereof, or a solvate thereof;
R₁ is selected from C₁₋₈ alkoxy, C₃₋₈ cycloalkoxy, C₂-C₈ heterocycloalkoxy, C₁-C₈ alkylthio, C₁-C₈ alkylsulfonyl, halo C₁₋₈ alkoxy, halo C₃₋₈ cycloalkoxy;
n is selected from 1.

More preferably, the compound of the present disclosure has a structure shown in general formula IV: 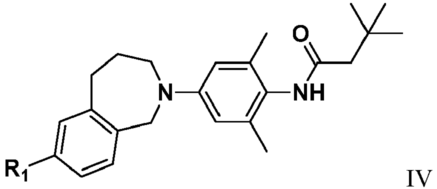
or a stereoisomer thereof, or a pharmaceutically acceptable salt thereof;
R₁ is selected from C₁₋₆ alkoxy, C₃₋₆ cycloalkoxy, C₂-C₆ heterocycloalkoxy, C₁-C₃ alkylthio, C₁-C₃ alkylsulfonyl, halo C₁₋₆ alkoxy, halo C₃₋₆ cycloalkoxy.

More preferably, the compound of the present disclosure has a structure shown in general formula IIc-2: 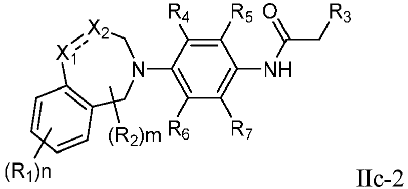
or a stereoisomer thereof, or a pharmaceutically acceptable salt thereof;
the bond between X₁ and X₂ is a double bond or a single bond;
n is selected from 2;
R₁ is selected from halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy.

More preferably, the compound of the present disclosure has a structure shown in general formula III: 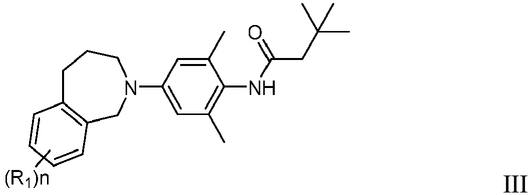
or a stereoisomer thereof, or a pharmaceutically acceptable salt thereof;
R₁ is selected from halogen, C₁₋₈ alkoxy, C₃₋₈ cycloalkoxy, C₂-C₈ heterocycloalkoxy, C₁-C₈ alkylthio, C₁-C₈ alkylsulfonyl, halo C₁₋₈ alkoxy, halo C₃₋₈ cycloalkoxy;
n is selected from 2.

More preferably, the compound of the present disclosure has a structure shown in general formula V: 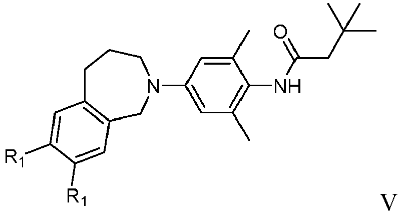
or a stereoisomer thereof, or a pharmaceutically acceptable salt thereof;
R₁ is selected from halogen, C₁₋₆ alkoxy, C₃₋₆ cycloalkoxy, C₂-C₆ heterocycloalkoxy, C₁-C₃ alkylthio, C₁-C₃ alkylsulfonyl, halo C₁₋₆ alkoxy, halo C₃₋₆ cycloalkoxy.

More preferably, the compound of the present disclosure has a structure shown in general formulas IIa to IIc or IIc-2:
X₁, X₂ are CH₂ with a single bond between them; R₄ and R₆ are H; R₅ and R₇ are methyl;
m is selected from 2, 3, 4, 5;
R₂ is not selected from hydrogen.
More preferably, the compound of the present disclosure has a structure shown in general formulas IIa to IIc or IIc-2:
R₆ is Cl; at least two of R₄, R₅, R₇ are not selected from hydrogen.

Preferably, the compound has a structure shown in general formula Va: 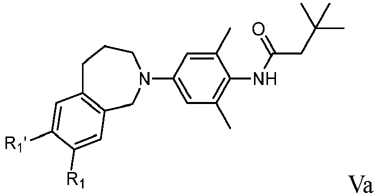
R₁' is H or F;
R₁ is

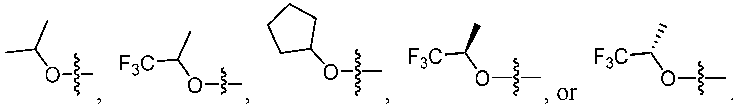

Preferably, the compound is selected from the following compounds, a stereoisomer thereof, a stereoisomer mixture thereof, or a pharmaceutically acceptable salt thereof:

| No. | Compound structure | No. | Compound structure |
|---|---|---|---|
| 001 | 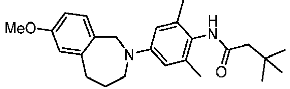 | 002 | 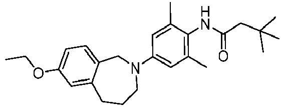 |
| 003 | 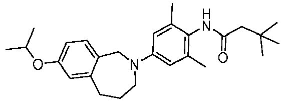 | 004 | 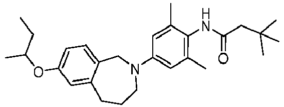 |
| 005 | 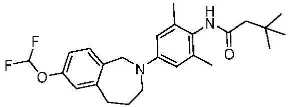 | 006 | 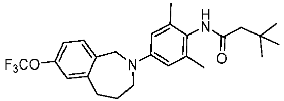 |
| 007 | 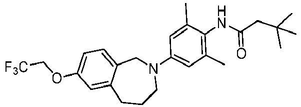 | 008 | 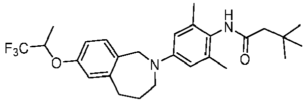 |
| 009 | 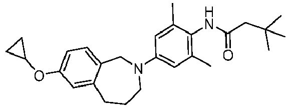 | 010 | 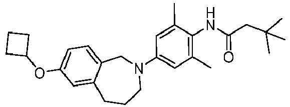 |
| 011 | 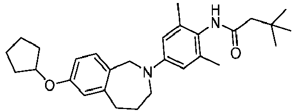 | 012 | 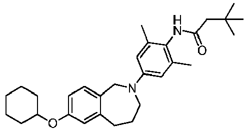 |
| 013 | 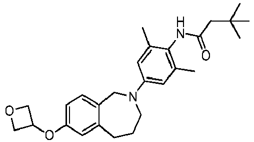 | 014 | 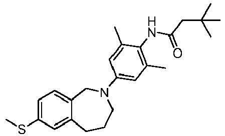 |
| 015 | 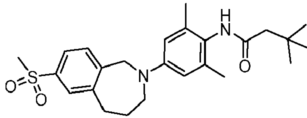 | 016 | 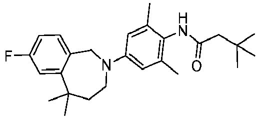 |
| 017 | 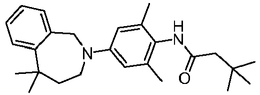 | 018 | 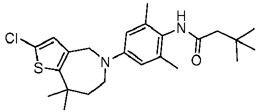 |
| 019 | 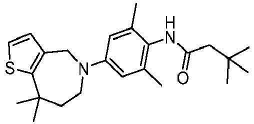 | 020 | 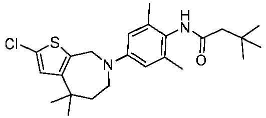 |
| 021 | 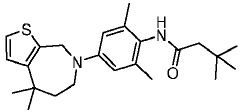 | 022 | 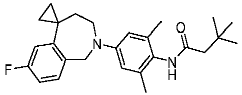 |
| 023 | 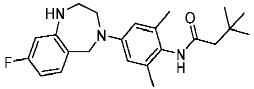 | 024 | 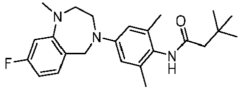 |
| 025 | 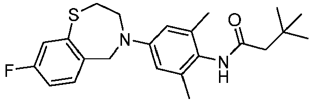 | 026 | 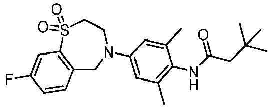 |
| 027 | 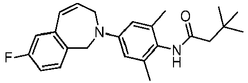 | 028 | 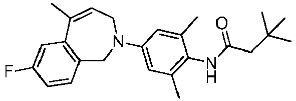 |
| 029 | 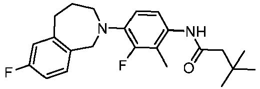 | 030 | 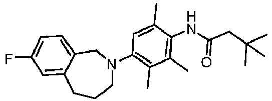 |
| 031 | 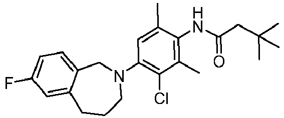 | 032 | 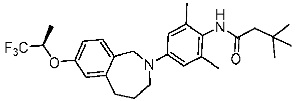 |
| 033 | 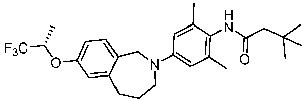 | 034 | 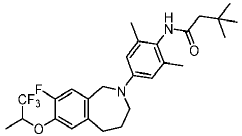 |
| 035 | 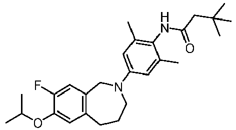 | 036 | 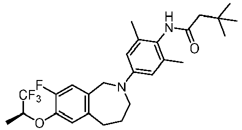 |
| 037 | 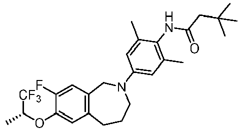 | 038 | 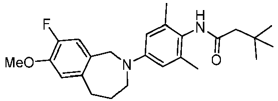 |
| 039 | 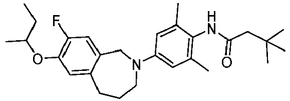 | 040 | 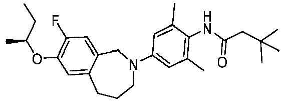 |
| 041 | 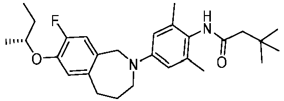 | 042 | 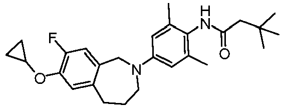 |
| 043 | 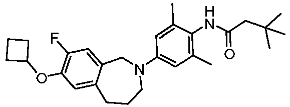 | 044 | 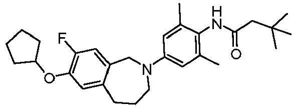 |
| 045 | 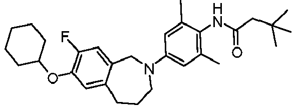 | 046 | 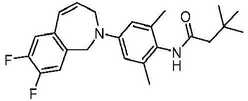 |
| 047 | 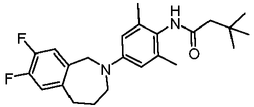 | 048 | 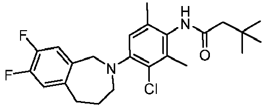 |
| 049 | 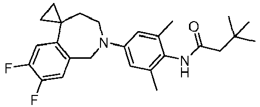 | 050 | 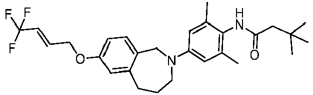 |
| 051 | 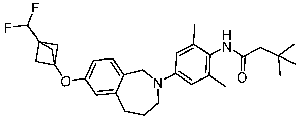 | 052 | 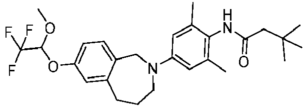 |
| 053 | 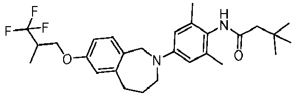 | 054 | 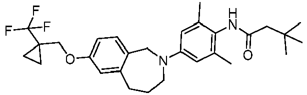 |
| 055 | 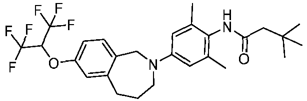 | 056 | 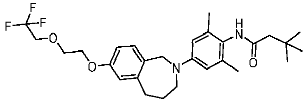 |
| 057 | 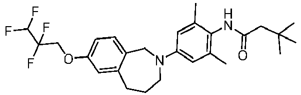 | 058 | 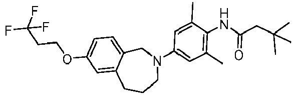 |
| 059 | 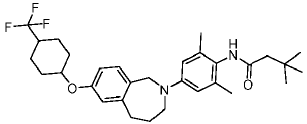 | 060 | 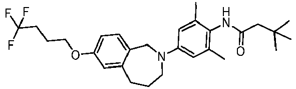 |
| 061 | 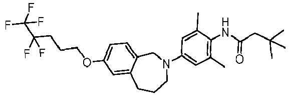 | 062 | 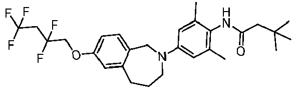 |
| 063 | 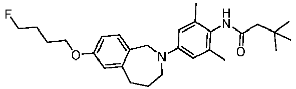 | | |

or a stereoisomer thereof, or a pharmaceutically acceptable salt thereof.

Preferably, the compound is the following compounds:
N-(4-(7-methoxy-1,3,4,5-tetrahydro-2H-benzo[c]azepin-2-yl)-2,6-dimethylphenyl)-3,3-dimethylbutanamide **(001)**
N-(4-(7-ethoxy-1,3,4,5-tetrahydro-2H-benzo[c]azepin-2-yl)-2,6-dimethylphenyl)-3,3-dimethylbutanamide **(002)**
N-(4-(7-isopropoxy-1,3,4,5-tetrahydro-2H-benzo[c]azepin-2-yl)-2,6-dimethylphenyl)-3,3-dimethylbutanamide **(003)**
N-(4-(7-(sec-butoxy)-1,3,4,5-tetrahydro-2H-benzo[c]azepin-2-yl)-2,6-dimethylphenyl)-3,3-dimethylbutanamide **(004)**
N-(4-(7-(difluoromethoxy)-1,3,4,5-tetrahydro-2H-benzo[c]azepin-2-yl)-2,6-dimethylphenyl)-3,3-dimethylbutanamide **(005)**
N-(2,6-dimethyl-4-(7-(trifluoromethoxy)-1,3,4,5-tetrahydro-2H-benzo[c]azepin-2-yl)phenyl)-3,3-dimethylbutanamide **(006)**
N-(2,6-dimethyl-4-(7-(2,2,2-trifluoroethoxy)-1,3,4,5-tetrahydro-2H-benzo[c]azepin-2-yl)phenyl)-3,3-dimethylbutanamide **(007)**
N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide **(008)**
N-(4-(7-cyclopropoxy-1,3,4,5-tetrahydro-2H-benzo[c]azepin-2-yl)-2,6-dimethylphenyl)-3,3-dimethylbutanamide **(009)**
N-(4-(7-cyclobutoxy-1,3,4,5-tetrahydro-2H-benzo[c]azepin-2-yl)-2,6-dimethylphenyl)-3,3-dimethylbutanamide **(010)**
N-(4-(7-(cyclopentyloxy)-1,3,4,5-tetrahydro-2H-benzo[c]azepin-2-yl)-2,6-dimethylphenyl)-3,3-dimethylbutanamide **(011)**
N-(4-(7-(cyclohexyloxy)-1,3,4,5-tetrahydro-2H-benzo[c]azepin-2-yl)-2,6-dimethylphenyl)-3,3-dimethylbutanamide **(012)**
N-(2,6-dimethyl-4-(7-(oxetan-3-yloxy)-1,3,4,5-tetrahydro-2H-benzo[c]azepin-2-yl)phenyl)-3,3-dimethylbutanamide **(013)**
N-(2,6-dimethyl-4-(7-(methylthio)-1,3,4,5-tetrahydro-2H-benzo[c]azepin-2-yl)phenyl)-3,3-dimethylbutanamide **(014)**
N-(2,6-dimethyl-4-(7-(methylsulfonyl)-1,3,4,5-tetrahydro-2H-benzo[c]azepin-2-yl)phenyl)-3,3-dimethylbutanamide **(015)**
N-(4-(7-fluoro-5,5-dimethyl-1,3,4,5-tetrahydro-2H-benzo[c]azepin-2-yl)-2,6-dimethylphenyl)-3,3-dimethylbutanamide **(016)**
N-(4-(5,5-dimethyl-1,3,4,5-tetrahydro-2H-benzo[c]azepin-2-yl)-2,6-dimethylphenyl)-3,3-dimethylbutanamide **(017)**
N-(4-(2-chloro-8,8-dimethyl-4,6,7,8-tetrahydro-5H-thieno[3,2-c]azepin-5-yl)-2,6-dimethylphenyl)-3,3-dimethylbutanamide **(018)**
N-(4-(8,8-dimethyl-4,6,7,8-tetrahydro-5H-thieno[3,2-c]azepin-5-yl)-2,6-dimethylphenyl)-3,3-dimethylbutanamide **(019)**
N-(4-(2-chloro-4,4-dimethyl-4,5,6,8-tetrahydro-7H-thieno[2,3-c]azepin-7-yl)-2,6-dimethylphenyl)-3,3-dimethylbutanamide **(020)**
N-(4-(4,4-dimethyl-4,5,6,8-tetrahydro-7H-thieno[2,3-c]azepin-7-yl)-2,6-dimethylphenyl)-3,3-dimethylbutanamide **(021)**
N-(4-(7-fluoro-3,4-dihydrospiro[benzo[c]azepine-5,1'-cyclopropan]-2(1H)-yl)-2,6-dimethylphenyl)-3,3-dimethylbutanamide **(022)**
N-(4-(8-fluoro-1,2,3,5-tetrahydro-4H-benzo[e][1,4]diazepin-4-yl)-2,6-dimethylphenyl)-3,3-dimethylbutanamide **(023)**
N-(4-(8-fluoro-1-methyl-1,2,3,5-tetrahydro-4H-benzo[e][1,4]diazepin-4-yl)-2,6-dimethylphenyl)-3,3-dimethylbutanamide **(024)**
N-(4-(8-fluoro-2,3-dihydrobenzo[f][1,4]thiazepin-4(5H)-yl)-2,6-dimethylphenyl)-3,3-dimethylbutanamide **(025)**
N-(4-(8-fluoro-1,1-dioxido-2,3-dihydrobenzo[f][1,4]thiazepin-4(5H)-yl)-2,6-dimethylphenyl)-3,3-dimethylbutanamide **(026)**
N-(4-(7-fluoro-1,3-dihydro-2H-benzo[c]azepin-2-yl)-2,6-dimethylphenyl)-3,3-dimethylbutanamide **(027)**
N-(4-(7-fluoro-5-methyl-1,3-dihydro-2H-benzo[c]azepin-2-yl)-2,6-dimethylphenyl)-3,3-dimethylbutanamide **(028)**
N-(3-fluoro-4-(7-fluoro-1,3,4,5-tetrahydro-2H-benzo[c]azepin-2-yl)-2-methylphenyl)-3,3-dimethylbutanamide **(029)**
N-(4-(7-fluoro-1,3,4,5-tetrahydro-2H-benzo[c]azepin-2-yl)-2,3,6-trimethylphenyl)-3,3-dimethylbutanamide **(030)**
N-(3-chloro-4-(7-fluoro-1,3,4,5-tetrahydro-2H-benzo[c]azepin-2-yl)-2,6-dimethylphenyl)-3,3-dimethylbutanamide **(031)**
(*R*)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide **(032)**
(*S*)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide **(033)**
(*R*)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide **(032)**
(*S*)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide **(033)**
N-(4-(8-fluoro-7-((1,1,1-trifluoropropan-2-yl)oxy)-1,3,4,5-tetrahydro-2H-benzo[c]azepin-2-yl)-2,6-dimethylphenyl)-3,3-dimethylbutanamide **(034)**
N-(4-(8-fluoro-7-isopropoxy-1,3,4,5-tetrahydro-2H-benzo[c]azepin-2-yl)-2,6-dimethylphenyl)-3,3-dimethylbutanamide **(035)**
(*S*)-N-(4-(8-fluoro-7-((1,1,1-tiifluoropropan-2-yl)oxy)-1,3,4,5-tetrahydro-2H-benzo[c]azepin-2-yl)-2,6-dimethylphenyl)-3,3-dimethylbutanamide **(036)**
(*R*)-N-(4-(8-fluoro-7-((1,1,1-trifluoropropan-2-yl)oxy)-1,3,4,5-tetrahydro-2H-benzo[c]azepin-2-yl)-2,6-dimethylphenyl)-3,3-dimethylbutanamide **(037)**
N-(4-(8-fluoro-7-methoxy-1,3,4,5-tetrahydro-2H-benzo[c]azepin-2-yl)-2,6-dimethylphenyl)-3,3-dimethylbutanamide **(038)**
N-(4-(7-(sec-butoxy)-8-fluoro-1,3,4,5-tetrahydro-2H-benzo[c]azepin-2-yl)-2,6-dimethylphenyl)-3,3-dimethylbutanamide **(039)**
(*S*)-N-(4-(7-(sec-butoxy)-8-fluoro-1,3,4,5-tetrahydro-2H-benzo[c]azepin-2-yl)-2,6-dimethylphenyl)-3,3-dimethylbutanamide **(040)**
(*R*)-N-(4-(7-(sec-butoxy)-8-fluoro-1,3,4,5-tetrahydro-2H-benzo[c]azepin-2-yl)-2,6-dimethylphenyl)-3,3-dimethylbutanamide **(041)**
N-(4-(7-cyclopropoxy-8-fluoro-1,3,4,5-tetrahydro-2H-benzo[c]azepin-2-yl)-2,6-dimethylphenyl)-3,3-dimethylbutanamide **(042)**
N-(4-(7-cyclobutoxy-8-fluoro-1,3,4,5-tetrahydro-2H-benzo[c]azepin-2-yl)-2,6-dimethylphenyl)-3,3-dimethylbutanamide **(043)**
N-(4-(7-(cyclopentyloxy)-8-fluoro-1,3,4,5-tetrahydro-2H-benzo[c]azepin-2-yl)-2,6-dimethylphenyl)-3,3-dimethylbutanamide **(044)**
N-(4-(7-(cyclohexyloxy)-8-fluoro-1,3,4,5-tetrahydro-2H-benzo[c]azepin-2-yl)-2,6-dimethylphenyl)-3,3-dimethylbutanamide **(045)**
N-(4-(7,8-difluoro-1,3-dihydro-2H-benzo[c]azepin-2-yl)-2,6-dimethylphenyl)-3,3-dimethylbutanamide **(046)**
N-(4-(7,8-difluoro-1,3,4,5-tetrahydro-2H-benzo[c]azepin-2-yl)-2,6-dimethylphenyl)-3,3-dimethylbutanamide **(047)**
N-(3-chloro-4-(7,8-difluoro-1,3,4,5-tetrahydro-2H-benzo[c]azepin-2-yl)-2,6-dimethylphenyl)-3,3-dimethylbutanamide **(048)**
N-(4-(7,8-difluoro-3,4-dihydrospiro[benzo[c]azepine-5,1'-cyclopropan]-2(1H)-yl)-2,6-dimethylphenyl)-3,3-dimethylbutanamide **(049)**
(*E*)-N-(2,6-dimethyl-4-(7-((4,4,4-trifluorobut-2-en-1-yl)oxy)-1,3,4,5-tetrahydro-2H-benzo[c]azepin-2-yl)phenyl)-3,3-dimethylbutanamide **(050)**
N-(4-(7-((3-(difluoromethyl)bicyclo[1.1.1]pentan-1-yl)oxy)-1,3,4,5-tetrahydro-2H-benzo[c]azepin-2-yl)-2,6-dimethylphenyl)-3,3-dimethylbutanamide **(051)**
N-(2,6-dimethyl-4-(7-(2,2,2-trifluoro-1-methoxyethoxy)-1,3,4,5-tetrahydro-2H-benzo[c]azepin-2-yl)phenyl)-3,3-dimethylbutanamide **(052)**
N-(2,6-dimethyl-4-(7-(3,3,3-trifluoro-2-methylpropoxy)-1,3,4,5-tetrahydro-2H-benzo[c]azepin-2-yl)phenyl)-3,3-dimethylbutanamide **(053)**
N-(2,6-dimethyl-4-(7-((1-(trifluoromethyl)cyclopropyl)methoxy)-1,3,4,5-tetrahydro-2H-benzo[c]azepin-2-yl)phenyl)-3,3-dimethylbutanamide **(054)**
N-(4-(7-((1,1,1,3,3,3-hexafluoropropan-2-yl)oxy)-1,3,4,5-tetrahydro-2H-benzo[c]azepin-2-yl)-2,6-dimethylphenyl)-3,3-dimethylbutanamide **(055)**
N-(2,6-dimethyl-4-(7-(2-(2,2,2-trifluoroethoxy)ethoxy)-1,3,4,5-tetrahydro-2H-benzo[c]azepin-2-yl)phenyl)-3,3-dimethylbutanamide **(056)**
N-(2,6-dimethyl-4-(7-(2,2,3,3-tetrafluoropropoxy)-1,3,4,5-tetrahydro-2H-benzo[c]azepin-2-yl)phenyl)-3,3-dimethylbutanamide **(057)**
N-(2,6-dimethyl-4-(7-(3,3,3-trifluoropropoxy)-1,3,4,5-tetrahydro-2H-benzo[c]azepin-2-yl)phenyl)-3,3-dimethylbutanamide **(058)**
N-(2,6-dimethyl-4-(7-((4-(trifluoromethyl)cyclohexyl)oxy)-1,3,4,5-tetrahydro-2H-benzo[c]azepin-2-yl)phenyl)-3,3-dimethylbutanamide **(059)**
N-(2,6-dimethyl-4-(7-(4,4,4-trifluorobutoxy)-1,3,4,5-tetrahydro-2H-benzo[c]azepin-2-yl)phenyl)-3,3-dimethylbutanamide **(060)**
N-(2,6-dimethyl-4-(7-((4,4,5,5,5-pentafluoropentyl)oxy)-1,3,4,5-tetrahydro-2H-benzo[c]azepin-2-yl)phenyl)-3,3-dimethylbutanamide **(061)**
N-(2,6-dimethyl-4-(7-(2,2,4,4,4-pentafluorobutoxy)-1,3,4,5-tetrahydro-2H-benzo[c]azepin-2-yl)phenyl)-3,3-dimethylbutanamide **(062)**
N-(4-(7-(4-fluorobutoxy)-1,3,4,5-tetrahydro-2H-benzo[c]azepin-2-yl)-2,6-dimethylphenyl)-3,3-dimethylbutanamide **(063)**

### Description of terms

Unless otherwise defined, all technical and scientific terms used herein have the same meaning as commonly understood by those skilled in the art. Unless otherwise specified, all patent documents, publicly disclosed materials, etc. referenced in the present disclosure are incorporated by reference in their entirety. If a term has multiple definitions in the present disclosure, the definition in this section shall prevail.

It should be understood that the general description in the foregoing and the detailed description in the following are only exemplary and explanatory and are not restrictive of any claims. It should be noted that, in the specification and the appended claims, unless otherwise stated, the singular forms such as "one", "an", "the" include plural forms. It should also be noted that, unless otherwise stated, "or" means "and/or". In addition, terms such as "contain" and "comprise" are not limiting.

"Substituted" means that a hydrogen atom is replaced by a substituent. It should be noted that the substituents on a specific atom are limited by its valence state. In the definition section, "Cᵢ-Cⱼ" and "Cᵢ-ⱼ" include the range from the starting point to the end point, wherein i and j are both integers representing the number of carbon atoms. For example, C₁-C₄, C₁-C₈, C₃-C₈, C₁₋₆, C₃₋₆.

C, H, O, S, N, F, Cl, Br, I, etc. involved in the groups and compounds of the present disclosure include their isotopes. At the same time, C, H, O, S, N, F, Cl, Br, and I involved in the groups and compounds of the present disclosure can optionally be substituted by one or more of their corresponding isotopes, including but not limited to carbon isotopes ¹²C, ¹³C, ¹⁴C, hydrogen isotopes protium (H), deuterium (D), tritium (T), oxygen isotopes ¹⁶O, ¹⁷O, ¹⁸O, sulfur isotopes ³²S, ³³S, ³⁴S, ³⁶S, nitrogen isotopes ¹⁴N, ¹⁵N, fluorine isotopes ¹⁷F, ¹⁹F, chlorine isotopes ³⁵Cl, ³⁷Cl, bromine isotopes ⁷⁹Br, ⁸¹Br, etc.

The term "alkyl" used in the present disclosure refers to a straight or branched saturated hydrocarbon group containing 1 to 8 carbon atoms, including but not limited to methyl, ethyl, propyl, isopropyl, n-butyl, isobutyl, sec-butyl, tert-butyl, n-pentyl, isopentyl, neopentyl, tert-pentyl, n-hexyl, isohexyl, neohexyl, heptyl, isoheptyl, neoheptyl, octyl, isooctyl, etc. Alkyl can be substituted by one or more than one substituent, and the substituents can be the same or different when the alkyl is substituted by more than one substituent; the substituent is independently D (deuterium), oxo, halogen, cyano, nitro, hydroxyl, amino, aminoalkyl, alkenyl, alkynyl, carboxyl, carboxylate, acyl, amido, methylsulfonyl, alkylamido, C₁-C₈ alkyl, C₁-C₈ alkoxy, C₁-C₈ hydroxyalkyl, C₁-C₈ alkylamino, halo C₁-C₈ alkyl, halo C₁-C₈ alkoxy, halo C₁-C₈ hydroxyalkyl, halo C₁-C₈ alkylamino, C₃-C₁₂ cycloalkyl, halo C₃-C₁₂ cycloalkyl, cycloalkenyl, cycloalkynyl, alkoxycarbonyl, alkylthio, alkylsulfonyl, hydroxyalkylamido, sulfonamido, spiroalkyl, C₆-C₁₂ aryl, C₅-C₁₄ heteroaryl, C₃-C₁₂ heterocyclyl.

The terms "alkenyl" and "chain alkenyl" used in the present disclosure refer to a straight or branched hydrocarbon chain group containing 1 to 8 carbon atoms and at least one C=C double bond, including but not limited to vinyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 1-methyl-2-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 1-methyl-2-hexenyl, 2-methyl-2-hexenyl, 2-methyl-3-hexenyl, 3,5-dimethyl-2-hexenyl, 3,3-dimethyl-1-pentenyl, 3-methyl-2-ethyl-1-butenyl, 1-octenyl, 2-octenyl, etc. Alkenyl can be substituted by one or more than one substituent, and the substituents can be the same or different when the alkenyl is substituted by more than one substituent; the substituent is independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido, spiroalkyl.

The term "alkynyl" used in the present disclosure refers to a straight or branched hydrocarbon chain group containing 1 to 8 carbon atoms and at least one C=C triple bond, including but not limited to ethynyl, 1-propynyl, 2-propynyl, 1-butynyl, 2-butynyl, 3-butynyl, 1-pentynyl, 2-pentynyl, 3-pentynyl, 4-pentynyl, 3-methyl-1-butynyl, 4-methyl-1-butynyl, 2-methyl-3-butynyl, 1-methyl-4-butynyl, 1-hexynyl, 2-hexynyl, 3-hexynyl, 4-hexynyl, 1-methyl-2-pentynyl, 1-methyl-3-pentynyl, 1-methyl-4-pentynyl, 2-methyl-3-pentynyl, 2,2-dimethyl-4-pentynyl, 1-heptynyl, 2-heptynyl, 3-heptynyl, 4-heptynyl, 5-heptynyl, 2-methyl-3-hexynyl, 3-methyl-1-hexynyl, 3,3-dimethyl-1-hexynyl, 4-methyl-1-hexynyl, etc. Alkynyl can be substituted by one or more than one substituent, and the substituents can be the same or different when the alkynyl is substituted by more than one substituent; the substituent is independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido, spiroalkyl.

The terms "halogen" and "halo" used in the present disclosure refer to fluorine, chlorine, bromine, and iodine, preferably fluorine, chlorine, and bromine.

The term "cycloalkyl" used in the present disclosure refers to a non-aromatic monovalent monocyclic or polycyclic (two monocyclic rings connected by chemical bonds, or bridged rings, or spiro rings, or fused rings) hydrocarbon group containing 3 to 12 carbon atoms, and one or more of the chemical bonds may be a double bond or a triple bond. "Cycloalkyl" includes, but is not limited to, cyclopropyl, cyclobutyl, cyclopentyl, cyclohexyl, cycloheptyl, cyclooctyl, cyclononyl, cyclodecyl, octahydroindene, decahydronaphthalene, bicyclo[1.1.0]butyl, bicyclo[2.1.0]pentyl, bicyclo[2.2.0]hexyl, bicyclo[3.2.0]heptyl, bicyclo[4.2.0]octyl, bicyclo[1.1.1]pentyl, bicyclo[2.1.1]hexyl, bicyclo[3.1.1]heptyl, bicyclo[2.2.1]heptyl, bicyclo[4.1.1]octyl, bicyclo[3.2.1]octyl, bicyclo[3.2.1]octyl, bicyclo[5.1.1]nonyl, bicyclo[4.2.1]nonyl, bicyclo[4.3.1]nonyl, bicyclo[3.2.2]nonyl, bicyclo[5.2.1]decyl, bicyclo[4.2.2]decyl, spiro[2.2]pentyl, spiro[2.3]hexyl, spiro[2.4]heptyl, spiro[2.5]octyl, spiro[2.6]nonyl, spiro[3.5]nonyl, spiro[3.4]octyl, spiro[3.3]heptyl, spiro[4.5]decyl, spiro[4.4]nonyl, etc. Cycloalkyl can be substituted by one or more than one substituent, and the substituents can be the same or different when the cycloalkyl is substituted by more than one substituent; the substituent is independently D (deuterium), oxo, halogen, cyano, nitro, hydroxyl, amino, aminoalkyl, alkenyl, alkynyl, carboxyl, carboxylate, acyl, amido, methylsulfonyl, alkylamido, C₁-C₈ alkyl, C₁-C₈ alkoxy, C₁-C₈ hydroxyalkyl, C₁-C₈ alkylamino, halo C₁-C₈ alkyl, halo C₁-C₈ alkoxy, halo C₁-C₈ hydroxyalkyl, halo C₁-C₈ alkylamino, C₃-C₁₂ cycloalkyl, halo C₃-C₁₂ cycloalkyl, cycloalkenyl, cycloalkynyl, alkoxycarbonyl, alkylthio, alkylsulfonyl, hydroxyalkylamido, sulfonamido, spiroalkyl, C₆-C₁₂ aryl, C₅-C₁₄ heteroaryl, C₃-C₁₂ heterocyclyl.

The term "cycloalkenyl" used in the present disclosure refers to a non-aromatic monovalent monocyclic or polycyclic (two monocyclic rings connected by chemical bonds, or bridged rings, or spiro rings, or fused rings) hydrocarbon group containing 3 to 12 carbon atoms and at least one C=C double bond, including but not limited to cyclopropenyl, cyclobutenyl, cyclopentenyl, cyclohexenyl, cycloheptenyl, cycloalkenyl, spiro[2.2]pent-1-enyl, spiro[2.2]pent-1,4-dienyl, spiro[2.3]hex-1-enyl, spiro[2.3]hex-1,4-dienyl, spiro[3.3]hept-1-enyl, spiro[3.3]hepta-1,5-dienyl, spiro[3.4]oct-1-enyl, spiro[3.4]oct-1,6-dienyl, spiro[3.4]oct-5-enyl, spiro[3.4]oct-6-enyl, spiro[3.4]oct-1-enyl, bicyclo[2.1.1]hex-1-enyl, bicyclo[2.1.1]hex-2-enyl, bicyclo[3.1.1]hept-1-enyl, bicyclo[3.1.1]hept-2-enyl, bicyclo[2.2.1]hept-1-enyl, bicyclo[2.2.1]hept-2-enyl, etc. Cycloalkene, cycloalkenyl can be substituted by one or more than one substituent, and the substituents can be the same or different when the cycloalkene or cycloalkenyl is substituted by more than one substituent; the substituent is independently D (deuterium), oxo, halogen, cyano, nitro, hydroxyl, aminoalkyl, alkenyl, alkynyl, carboxyl, carboxylate, acyl, amido, methylsulfonyl, alkylamido, C₁-C₈ alkyl, C₁-C₈ alkoxy, C₁-C₈ hydroxyalkyl, C₁-C₈ alkylamino, halo C₁-C₈ alkyl, halo C₁-C₈ alkoxy, halo C₁-C₈ hydroxyalkyl, halo C₁-C₈ alkylamino, C₃-C₁₂ cycloalkyl, halo C₃-C₁₂ cycloalkyl, cycloalkenyl, cycloalkynyl, alkoxycarbonyl, alkylthio, alkylsulfonyl, hydroxyalkylamido, sulfonamido, spiroalkyl, C₆-C₁₂ aryl, C₅-C₁₂ heteroaryl, C₃-C₁₂ heterocyclyl.

The terms "heterocyclyl" and "heterocycloalkyl" used in the present disclosure refer to a monocyclic or polycyclic (two monocyclic rings connected by chemical bonds, or bridging rings, or spiro rings, or fused rings) saturated or unsaturated (with one or more double bonds, conjugated, or partly conjugated) divalent cyclic hydrocarbon group with 3 to 15 ring atoms, with one or more than one heteroatom selected from N, O, and S. Heterocyclyl includes, but is not limited to, pyridyl, pyrimidyl, pyrazinyl, pyridazinyl, triazinyl, isoxazolyl, isothiazolyl, pyrazolyl, thiazolyl, thienyl, furyl, triazolyl, oxazolyl, imidazolyl, indazolyl, indolizinyl, indolyl, indolinyl, isoindolinyl, quinolinyl, isoquinolinyl, quinoxalinyl, quinazolinyl, dihydroquinolinyl, tetrahydroquinolinyl, dihydroisoquinolinyl, dihydroisoquinolinyl, tetrahydroisoquinolinyl, cinnolinyl, pteridinyl, purinyl, pyranyl, piperidinyl, piperazinyl, morpholinyl, dioxanyl, oxiranyl, oxetanyl, oxanyl, oxepanyl, oxocanyl, aziridinyl, azetidinyl, azepanyl, azocanyl, azacyclooctyl, thietanyl, thiiranyl, azacyclooctyl, oxazepinyl, diazepinyl, thiazepinyl, dihydrofuryl, dihydrothiophenyl, dihydropyranyl, tetrahydrofuryl, tetrahydrothiophenyl, tetrahydropyranyl, tetrahydrothiopyranyl, tetrahydrothiazolyl, tetrahydroimidazolyl, hexahydropyridazinyl, hexahydropyrimidinyl, thienoazepine, benzazepine, benzimidazolyl, benzofuranyl, benzothienyl, benzothiazolyl, benzotriazolyl, benzotriazinyl, benzoxadiazolyl, benzoxazolyl, benzisoxazolyl, imidazopyridyl, imidazothiazolyl, pyrrolopyridyl, thienopyrrolyl, thienothienyl, thienopyridyl, thienopyrimidinyl, pyrazolopyrimidinyl, pyrrolopyrrolyl, pyrrolopyrimidinyl, pyrrolopyridazinyl, 1-azaspiro[2.2]pentyl, 1-azaspiro[2.3]hexyl, 4-azaspiro[2.3]hexyl, 5-azaspiro[2.3]hexyl, 2-azaspiro[3.3]heptyl, 2,6-diazaspiro[3.3]heptyl, 2-oxa-6-azaspiro[3.3]heptyl, 1-azaspiro[2.5]octyl, 2-azaspiro[3.4]octyl, 6-azaspiro[3.4]octyl, 2,6-diazaspiro[3.4]octyl, 2-azaspiro[3.5]nonyl, 6-azaspiro[3.4]octyl, 6-azaspiro[3.4]octyl, 2-azaspiro[3.5]nonyl, 6-azaspiro[3.5]nonyl, 7-azaspiro[3.5]nonyl, 2,7-diazaspiro[3.5]nonyl, 2-oxa-7-azaspiro[3.5]nonyl, 1-azaspiro[4.4]nonyl, 2-azaspiro[4.4]nonyl, 8-azaspiro[4.5]decyl, 2,8-diazaspiro[4.5]decyl, 1-oxaspiro[2.2]pentyl, 1-oxaspiro[2.3]hexyl, 4-oxaspiro[2.3]hexyl, 5-oxaspiro[2.3]hexyl, 2-oxaspiro[3.3]heptyl, 1-oxaspiro[2.5]octyl, 2-oxaspiro[3.4]octyl, 6-oxaspiro[3.4]octyl, 2 -oxaspiro[3.5]nonyl, 6-oxaspiro[3.5]nonyl, 7-oxaspiro[3.5]nonyl, 1-oxaspiro[4.4]nonyl, 2-oxaspiro[4.4]nonyl, 8-oxaspiro[4.5]decanyl, decahydroquinolinyl, decahydroisoquinolinyl, 2-azabicyclo[1.1.1]pentyl, 2-oxabicyclo[1.1.1]pentyl, azabicyclo[2.1.1]hexyl, oxabicyclo[2.1.l]hexyl, azabicyclo[3.1.1]heptyl, oxabicyclo[3.1.1]heptyl, azabicyclo[2.2.1]heptyl, azabicyclo[4.1.1]octyl, azabicyclo[3.2.1]octyl, azabicyclo[3.2.1]octyl, etc. Heterocyclyl can be substituted by one or more than one substituent, and the substituents can be the same or different when the heterocyclyl is substituted by more than one substituent; the substituent is independently D (deuterium), oxo, halogen, cyano, nitro, hydroxyl, amino, aminoalkyl, alkenyl, alkynyl, carboxyl, carboxylate, acyl, amido, methylsulfonyl, alkylamido, C₁-C₈ alkyl, C₁-C₈ alkoxy, C₁-C₈ hydroxyalkyl, C₁-C₈ alkylamino, halo C₁-C₈ alkyl, halo C₁-C₈ alkoxy, halo C₁-C₈ hydroxyalkyl, halo C₁-C₈ alkylamino, C₃-C₁₂ cycloalkyl, halo C₃-C₁₂ cycloalkyl, alkoxycarbonyl, alkylthio, alkylsulfonyl, hydroxyalkylamido, sulfonamido, spiroalkyl, C₆-C₁₂ aryl, C₅-C₁₄ heteroaryl, C₃-C₁₂ heterocyclyl. As defined herein, the term "heterocyclyl" may include aromatic heterocyclyl or non-aromatic heterocyclyl or aliphatic heterocyclyl having at least one heteroatom.

The term "spiroalkyl" used in the present disclosure means that two carbon atoms of an alkyl group are connected to the same carbon atom of the parent molecular group, thereby forming a 3- to 12-membered carbocyclic ring.

The term "alkoxy" used in the present disclosure refers to alkyl-O-, wherein the alkyl is as defined above. Examples of "alkoxy" used in the present disclosure include, but are not limited to, methoxy, ethoxy, n-propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, etc. "Alkoxy" also includes substituted alkoxy whose substituents can be D, halogen, oxo, amino, hydroxyl, cyano, nitro, carboxyl, amido, sulfonamido, spiroalkyl, C₁-C₈ alkyl, C₁-C₈ hydroxyalkyl, C₁-C₈ alkoxy, C₁-C₈ alkylamino, halo C₁-C₈ alkyl, halo C₁-C₈ hydroxyalkyl, halo C₁-C₈ alkoxy, halo C₁-C₈ alkylamino, C₃-C₁₂ cycloalkyl, C₃-C₁₂ heterocyclyl, C₆-C₁₂ aryl, C₅-C₁₄ heteroaryl.

The term "alkenoxy" used in the present disclosure refers to alkenyl-O-, wherein alkenyl is as defined above. "Alkenoxy" also includes substituted alkenoxy whose substituents can be D, halogen, oxo, amino, hydroxyl, cyano, nitro, carboxyl, amido, sulfonamido, spiroalkyl, C₁-C₈ alkyl, C₁-C₈ hydroxyalkyl, C₁-C₈ alkoxy, C₁-C₈ alkylamino, halo C₁-C₈ alkyl, halo C₁-C₈ hydroxyalkyl, halo C₁-C₈ alkoxy, halo C₁-C₈ alkylamino, C₃-C₁₂ cycloalkyl, C₃-C₁₂ heterocyclyl, C₆-C₁₂ aryl, C₅-C₁₄ heteroaryl.

The term "cycloalkoxy" used in the present disclosure refers to cycloalkyl-O-, wherein cycloalkyl is as defined above. Examples of "cycloalkoxy" used in the present disclosure include, but are not limited to, cyclopropyloxy, cyclobutyloxy, cyclopentyloxy, cyclohexyloxy, cycloheptyloxy, cyclooctyloxy, bicyclo[1.1.0]butyloxy, bicyclo[2.1.0]pentyloxy, bicyclo[2.2.0]hexyloxy, bicyclo[1.1.1]pentyloxy, bicyclo[2.1.1]hexyloxy, etc. "Cycloalkoxy" also includes substituted cycloalkoxy whose substituents can be D, halogen, oxo, amino, hydroxyl, cyano, nitro, carboxyl, amido, sulfonamido, spiroalkyl, C₁-C₈ alkyl, C₁-C₈ hydroxyalkyl, C₁-C₈ alkoxy, C₁-C₈ alkylamino, halo C₁-C₈ alkyl, halo C₁-C₈ hydroxyalkyl, halo C₁-C₈ alkoxy, halo C₁-C₈ alkylamino, C₃-C₁₂ cycloalkyl, C₃-C₁₂ heterocyclyl, C₆-C₁₂ aryl, C₅-C₁₄ heteroaryl.

The term "heterocycloalkoxy" used in the present disclosure refers to heterocycloalkyl-O-, wherein heterocycloalkyl is as defined above. Examples of "heterocycloalkoxy" used in the present disclosure include, but are not limited to, oxiranyloxy, oxetanyloxy, oxolanyloxy, oxanyloxy, aziridinyloxy, azetidinyloxy, pyrrolidinyloxy, piperidinyloxy, etc. "Heterocycloalkoxy" also includes substituted heterocycloalkoxy whose substituents can be D, halogen, oxo, amino, hydroxyl, cyano, nitro, carboxyl, amido, sulfonamido, spiroalkyl, C₁-C₈ alkyl, C₁-C₈ hydroxyalkyl, C₁-C₈ alkoxy, C₁-C₈ alkylamino, halo C₁-C₈ alkyl, halo C₁-C₈ hydroxyalkyl, halo C₁-C₈ alkoxy, halo C₁-C₈ alkylamino, C₃-C₁₂ cycloalkyl, C₃-C₁₂ heterocyclyl, C₆-C₁₂ aryl, C₅-C₁₄ heteroaryl.

The term "haloalkyl" used in the present disclosure refers to a straight or branched alkyl substituted by halogen (preferably fluorine, chlorine, bromine, iodine), wherein "alkyl" is as defined above. Examples of "haloalkyl" used in the present disclosure include, but are not limited to, fluoromethyl, difluoromethyl, trifluoromethyl, chloromethyl, dichloromethyl, trichloromethyl, 2,2,2-trifluoroethyl, 2-fluoroethyl, 2-chloroethyl, tetrafluoroethyl, pentafluoroethyl, 1,1,1-trifluoropropan-2-yl, etc. "Haloalkyl" also includes substituted haloalkyl whose substituents can be D, oxo, amino, hydroxyl, cyano, nitro, carboxyl, amido, spiroalkyl, C₁-C₈ alkyl, C₁-C₈ hydroxyalkyl, C₁-C₈ alkoxy, C₁-C₈ alkylamino, C₃-C₁₂ cycloalkyl, C₃-C₁₂ heterocyclyl, C₆-C₁₂ aryl, C₅-C₁₄ heteroaryl. The halogen substituent in "haloalkyl" can be one or more, which may be substituted on one atom or on different atoms.

The term "haloalkoxy" used in the present disclosure refers to haloalkyl-O-, wherein haloalkyl is as defined above. Examples of "haloalkoxy" used in the present disclosure, include but are not limited to, monofluoromethoxy, difluoromethoxy, trifluoromethoxy, monofluoroethoxy, difluoroethoxy, trifluoroethoxy, monofluoropropoxy, difluoropropoxy, trifluoropropoxy, tetrafluoropropoxy, trifluoroisopropoxy, tetrafluoroisopropoxy, hexafluoroisopropoxy, monofluorobutoxy, difluorobutoxy, trifluorobutoxy, trifluoro-sec-butoxy, trifluoro-tert-butoxy, trifluoroisobutoxy, hexafluoroisobutoxy, trifluoropentyloxy, tetrafluoropentyloxy, pentafluoropentyloxy, trifluoroisopentyloxy, monochloromethoxy, dichloromethoxy, monochloroethoxy, dichloroethyl, chloropropyl, etc. "Haloalkoxy" also includes substituted haloalkoxy whose substituents can be D, oxo, amino, hydroxyl, cyano, nitro, carboxyl, amido, spiroalkyl, C₁-C₈ alkyl, C₁-C₈ hydroxyalkyl, C₁-C₈ alkoxy, C₁-C₈ alkylamino, C₃-C₁₂ cycloalkyl, C₃-C₁₂ heterocyclyl, C₆-C₁₂ aryl, C₅-C₁₄ heteroaryl.

The term "halocycloalkyl" used in the present disclosure refers to a cycloalkyl group substituted by halogen (preferably fluorine, chlorine, bromine, iodine), wherein cycloalkyl is as defined above. The halogen substituents in "halocycloalkyl" can be one or more, which may be substituted on one atom or on different atoms.

The term "halocycloalkoxy" used in the present disclosure refers to halocycloalkyl-O-, wherein halocycloalkyl is as defined above. "Halocycloalkoxy" also includes substituted halocycloalkoxy whose substituents can be D, oxo, amino, hydroxyl, cyano, nitro, carboxyl, amido, spiroalkyl, C₁-C₈ alkyl, C₁-C₈ hydroxyalkyl, C₁-C₈ alkoxy, C₁-C₈ alkylamino, C₃-C₁₂ cycloalkyl, C₃-C₁₂ heterocyclyl, C₆-C₁₂ aryl, C₅-C₁₄ heteroaryl.

The term "haloheterocycloalkyl" used in the present disclosure refers to a heterocycloalkyl group substituted by halogen (preferably fluorine, chlorine, bromine, iodine), wherein the heterocycloalkyl is as defined above. The halogen substituents in "haloheterocycloalkyl" can be one or more, which may be substituted on one atom or on different atoms.

The term "haloheterocycloalkoxy" used in the present disclosure refers to haloheterocycloalkyl-O-, wherein haloheterocycloalkyl is as defined above. "Haloheterocycloalkoxy" also includes substituted haloheterocycloalkoxy whose substituents can be D, oxo, amino, hydroxyl, cyano, nitro, carboxyl, amido, spiroalkyl, C₁-C₈ alkyl, C₁-C₈ hydroxyalkyl, C₁-C₈ alkoxy, C₁-C₈ alkylamino, C₃-C₁₂ cycloalkyl, C₃-C₁₂ heterocyclyl, C₆-C₁₂ aryl, C₅-C₁₄ heteroaryl.

The term "alkylthio" used in the present disclosure refers to alkyl-S-, wherein alkyl is as defined above. Alkylthio includes, but is not limited to, methylthio, ethylthio, propylthio, butylthio, etc.

The term "alkanoyl" used in the present disclosure refers to alkyl-C(O)-, wherein alkyl is as defined above.

The term "alkylsulfonyl" used in the present disclosure refers to alkyl-S(O)₂-, wherein alkyl is as defined above.

The term "aminosulfonyl" used in the present disclosure refers to amino-S(O)₂-.

"Pharmaceutically acceptable salt" refers to salts of the compounds of the present disclosure that can be prepared by methods well known to those skilled in the art. The salt may be a salt formed with an acid or a base, etc. A preferred class of salts are the salts of the compounds of the present disclosure formed with acids. Acids suitable for forming salts include, but are not limited to, inorganic acids such as hydrochloric acid, hydrobromic acid, hydrofluoric acid, hydroiodic acid, sulfuric acid, nitric acid, phosphoric acid, carbonic acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, trifluoromethanesulfonic acid, citric acid, p-toluenesulfonic acid, ethanesulfonic acid, benzenesulfonic acid, camphorsulfonic acid, ascorbic acid 2-glucoside, isonicotinic acid, salicylic acid, ascorbic acid, gentisic acid, gluconic acid, pyruvic acid, naphthalenesulfonic acid, stearic acid, phenylacetic acid, p-aminobenzenesulfonic acid, 2-hydroxyethanesulphonic acid, pamoic acid, and tannic acid; and acidic amino acids such as aspartic acid and glutamic acid. A preferred class of salts are the salts of the compounds of the present disclosure formed with bases. Bases suitable for forming salts, include but are not limited to: inorganic bases such as sodium hydroxide, potassium hydroxide, sodium carbonate, sodium bicarbonate, sodium phosphate; organic bases such as ammonia water, triethylamine, diethylamine, piperazine, guanidine, diethanolamine.

The second object of the present disclosure is to provide a pharmaceutical composition comprising one or more than one compound described in any one of the above technical solutions. The pharmaceutical composition of the present disclosure can be composed of one or more than one compound described in any one of the above technical solutions and other compounds, or one or more than one compound described in any of the above technical solutions.

Another object of the present disclosure is to provide a pharmaceutical formulation comprising one or more than one compound described in any one of the above technical solutions. Preferably, tablets, powders, capsules, injections, granules, and sprays are included. On the other hand, the present disclosure provides the compounds of general formulas I to V, the stereoisomer thereof, the stereoisomer mixture thereof, or the pharmaceutically acceptable salt thereof disclosed herein for use in the treatment of diseases, disorders, or conditions benefiting from the activation of KCNQ.

On the other hand, the present disclosure provides a use of the compounds of general formulas I to V disclosed herein in the manufacture of a medicament for diseases that are sensitive to the increase of potassium channel ion flow, especially in the manufacture of a medicament for the treatment of central nervous system diseases.

In some embodiments, the subject in need thereof suffers from cancer including epilepsy, inflammatory pain, neuropathic pain, migraine, neurodegenerative disease, anxiety disorders, stroke, complications caused by cocaine abuse, nicotine withdrawal syndrome, alcohol withdrawal syndrome, or tinnitus.

The inventors of the present disclosure have confirmed through experiments that the compounds of the present disclosure have an obvious activation effect on KCNQ2/3 potassium ions.

The inventors of the present disclosure have confirmed through experiments that the compounds of the present disclosure have the effect of significantly inducing a left shift of the half-maximal activation voltage.

The inventors of the present disclosure have confirmed through experiments that the compounds of the present disclosure can significantly inhibit the occurrence of hind limb tonic extension in MES-induced epilepsy in mice.

The inventors of the present disclosure have confirmed through experiments that the compounds of the present disclosure have better intracerebral exposure.

### DETAILED DESCRIPTION OF THE PREFERRED EMBODIMENT

The implementability of the present disclosure is illustrated by the following examples. Those skilled in the art should understand that the corresponding technical features modified or replaced according to the teaching of the prior art still fall within the protection scope of the present disclosure.

### Example 1: N-(4-(7-methoxy-1,3,4,5-tetrahydro-2H-benzo[c]azepin-2-yl)-2,6-dimethylphenyl)-3,3-dimethylbutanamide (001)

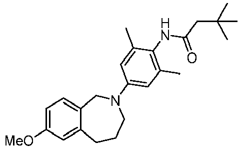

Step 1, 6-hydroxy-1-tetralone (1 g, 6.17 mmol) was dissolved in 10 mL of DMF, and the mixture was added with cesium carbonate (4.02 g, 12.33 mmol), added with iodomethane (1.31 g, 9.25 mmol) at 0°C, and reacted at 80°C for 2 hours. After the reaction was completed, the reaction mixture was cooled to room temperature, added with 30 mL of water, and extracted with ethyl acetate. The organic phase was washed twice with saturated sodium bicarbonate aqueous solution, and concentrated under reduced pressure to precipitate a large amount of white solids. The residue was filtered under reduced pressure, and the filter cake was dried to obtain the crude product, which was purified by silica gel column chromatography to obtain intermediate 001-1: 6-methoxy-1-tetralone (1.06 g) with a yield of 96%. LC-MS (ESI-MS): 177[M+H]⁺. The intermediate 001-1 was used directly in the next step.

Step 2, intermediate 001-1 (1 g, 5.67 mmol) was dissolved in 10 mL of concentrated hydrochloric acid, and the mixture was slowly added with sodium azide (737.86 mg, 11.35 mmol) at 0°C, gradually warmed to room temperature, and reacted for 6 hours. After the reaction was completed, the reaction mixture was slowly added with saturated potassium carbonate aqueous solution to adjust the pH of the system around 8, and extracted with ethyl acetate. The organic phases were combined, dried over anhydrous sodium sulfate, concentrated under reduced pressure, and subjected to silica gel column chromatography to obtain intermediate 001-2: 7-methoxy-2,3,4,5-tetrahydro-1H-benzo[c]azepin-1-one (0.52 mg) with a yield of 48%. LC-MS (ESI-MS): 192[M+H]⁺.

Step 3, intermediate 001-2 (250 mg, 1.31 mmol) was dissolved in 1 mL of tetrahydrofuran, and the mixture was slowly added dropwise with lithium aluminum hydride (2.6 mL, 5.23 mmol, 2M in THF) at 0°C, heated to reflux and reacted for 4 hours. After the reaction was completed, the reaction mixture was slowly added dropwise with water until the system stopped bubbling, and then concentrated under reduced pressure. The residue was dissolved with ethyl acetate and concentrated under reduced pressure to obtain intermediate 001-3: 7-methoxy-2,3,4,5-tetrahydro-1H-benzo[c]azepine (200 mg) with a yield of 86%. LC-MS (ESI-MS): 178[M+H]⁺.

Step 4, intermediate 001-3 (100 mg, 0.56 mmol) was dissolved in 2 mL of 1,4-dioxane, and the mixture was sequentially added with N-(2,6-dimethyl-4-bromophenyl)-3,3-dimethylbutanamide (252.38 mg, 0.84 mmol), Pd₂(dba)₃ (25.83 mg, 0.02 mmol), XPhos (2-dicyclohexylphosphino-2',4',6'-triisopropylbiphenyl) (26.90 mg, 0.05 mmol), and sodium tert-butoxide (162.66 mg, 1.69 mmol), replaced with nitrogen, and reacted at 80°C for 2 hours. After the reaction was completed, the reaction mixture was cooled to room temperature and filtered under reduced pressure. The solid residue was washed with ethyl acetate, and then washed three times with saturated ammonium chloride solution. The organic phase was concentrated and subjected to silica gel column chromatography to obtain the target compound 001 (90 mg) with ayield of 40%. LC-MS (ESI-MS): 395[M+H]⁺, ¹H NMR (400 MHz, CDCl₃) δ 7.18 (d, *J* = 8.2 Hz, 1H), 6.67 (d, *J* = 2.4 Hz, 1H), 6.62 (dd, *J* = 8.1, 2.6 Hz, 1H), 6.48 (s, 2H), 6.40 (brs, 1H), 4.49 (s, 2H), 3.75 (s, 3H), 3.73 - 3.70 (m, 2H), 2.95 - 2.88 (m, 2H), 2.23 (s, 2H), 2.15 (s, 6H), 1.88 - 1.82 (m, 2H), 1.12 (s, 9H).

### Example 2: N-(4-(7-ethoxy-1,3,4,5-tetrahydro-2H-benzo[c]azepin-2-yl)-2,6-dimethylphenyl)-3,3-dimethylbutanamide (002)

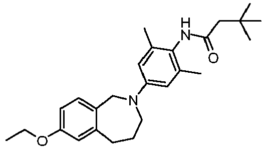

With reference to the synthetic route and method of Example 1, iodomethane was replaced by bromoethane to obtain the target compound 002 (35 mg). LC-MS (ESI-MS): 409[M+H]⁺, ¹H NMR (400 MHz, CDCl₃) δ 7.16 (d, *J* = 8.2 Hz, 1H), 6.66 (d, *J* = 2.5 Hz, 1H), 6.61 (dd, *J* = 8.2, 2.6 Hz, 1H), 6.49 (d, *J* = 6.1 Hz, 2H), 6.39 (brs, 1H), 4.48 (s, 2H), 3.97 (q, *J* = 7.0 Hz, 2H), 3.73 - 3.68 (m, 2H), 2.93 - 2.87 (m, 2H), 2.23 (s, 2H), 2.14 (s, 6H), 1.88 - 1.82 (m, 2H), 1.37 (t, *J* = 7.0 Hz, 3H), 1.12 (s, 9H).

### Example 3: N-(4-(7-isopropoxy-1,3,4,5-tetrahydro-2H-benzo[c]azepin-2-yl)-2,6-dimethylphenyl)-3,3-dimethylbutanamide (003)

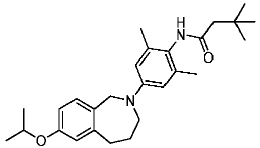

With reference to the synthetic route and method of Example 1, iodomethane was replaced by 2-bromopropane to obtain the target compound 003 (46 mg). LC-MS (ESI-MS): 423 [M+H]⁺, ¹H NMR (400 MHz, CDCl₃) δ 7.15 (d, *J* = 8.2 Hz, 1H), 6.65 (d, *J* = 2.4 Hz, 1H), 6.60 (dd, *J* = 8.2, 2.5 Hz, 1H), 6.48 (s, 2H), 6.42 (brs, 1H), 4.51 - 4.44 (m, 3H), 3.71 (d, *J* = 4.6 Hz, 2H), 2.92 - 2.85 (m, 2H), 2.23 (s, 2H), 2.14 (s, 6H), 1.85 (d, *J* = 4.3 Hz, 2H), 1.30 (d, *J* = 6.1 Hz, 6H), 1.12 (s, 9H).

### Example 4: N-(4-(7-(sec-butoxy)-1,3,4,5-tetrahydro-2H-benzo[c]azepin-2-yl)-2,6-dimethylphenyl)-3,3-dimethylbutanamide (004)

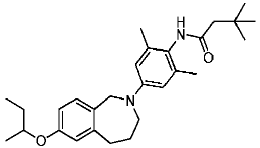

With reference to the synthetic route and method of Example 1, iodomethane was replaced by 2-bromobutane to obtain the target compound 004 (60 mg). LC-MS (ESI-MS): 437[M+H]⁺,¹H NMR (400 MHz, CDCl₃) δ 7.15 (d, *J* = 8.2 Hz, 1H), 6.65 (d, *J* = 2.4 Hz, 1H), 6.60 (dd, *J* = 8.2, 2.5 Hz, 1H), 6.49 (d, *J* = 7.5 Hz, 2H), 6.42 (brs, 1H), 4.49 (s, 2H), 4.22 (dd, *J* = 12.1, 6.1 Hz, 1H), 3.75 - 3.68 (m, 2H), 2.93 - 2.86 (m, 2H), 2.23 (s, 2H), 2.15 (s, 6H), 1.88 - 1.80 (m, 2H), 1.75 - 1.68 (m, 2H), 1.25 (s, 3H), 1.12 (s, 9H), 0.95 - 0.92 (m, 3H).

### Example 5: N-(4-(7-(difluoromethoxy)-1,3,4,5-tetrahydro-2H-benzo[c]azepin-2-yl)-2,6-dimethylphenyl)-3,3-dimethylbutanamide (005)

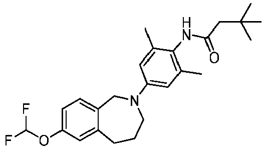

Step 1, 6-hydroxy-1-tetralone (2 g, 12.33 mmol) was dissolved in 10 mL of DMF, and the mixture was sequentially added with potassium iodide (2.07 g, 12.45 mmol), tetrabutylammonium iodide (4.78 g, 12.45 mmol), sodium bromodifluoroacetate (2.45 g, 12.45 mmol), and cesium carbonate (8.04 g, 24.66 mmol), and reacted overnight at 100°C. After the reaction was completed, the reaction mixture was cooled to room temperature, diluted with ethyl acetate, and filtered under reduced pressure. The organic phase was washed twice with saturated sodium bicarbonate, twice with saturated ammonium chloride, concentrated under reduced pressure, and subjected to silica gel column chromatography to obtain the product 6-(difluoromethoxy)-1-tetralone (1.4 g) with a yield of 53%. LC-MS (ESI-MS): 213[M+H]⁺.

The synthetic route and method of Example 1 were referenced in the subsequent steps, and 6-methoxy-1-tetralone was replaced by 6-(difluoromethoxy)-1-tetralone (1.4 g) to obtain the target compound 005 (28 mg), LC-MS (ESI-MS): 431[M+H]⁺. ¹H NMR (400 MHz, CDCl₃) δ 7.24 (d, *J* = 8.2 Hz, 1H), 6.90 - 6.83 (m, 2H), 6.47 (s, 2H), 6.44 (s, 1H), 6.41 (s, 1H), 4.53 (s, 2H), 3.77 - 3.71 (m, 2H), 2.97 - 2.91 (m, 2H), 2.23 (s, 2H), 2.15 (s, 6H), 1.86 (dd, *J* = 9.9, 5.7 Hz, 2H), 1.12 (s, 9H).

### Example 6: N-(2,6-dimethyl-4-(7-(trifluoromethoxy)-1,3,4,5-tetrahydro-2H-benzo[c]azepin-2-yl)dimethylphenyl)-3,3-dithylbutanamide (006)

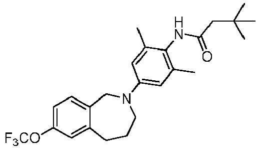

Step 1, 6-hydroxy-1-tetralone (0.8 g, 5 mmol) was dissolved in 10 mL of anhydrous DMF, and the mixture was added with potassium carbonate (0.83 g, 6 mmol) and S-(trifluoromethyl)dibenzothiophenium trifluoromethanesulfonate (2.4 g, 6 mmol), and stirred at room temperature for about 12 hours. After the reaction was completed, the reaction mixture was added with water and ethyl acetate for extraction. The organic phase was washed with saturated brine, dried over anhydrous sodium sulfate, concentrated under reduced pressure, and purified by silica gel column chromatography to obtain the product 6-(trifluoromethoxy)-1-tetralone (0.52 g) with a yield of 45%, LC-MS (ESI-MS): 231[M+H]⁺.

The synthetic route and method of Example 1 were referenced in the subsequent steps, and 6-methoxy-1-tetralone was replaced by 6-(trifluoromethoxy)-1-tetralone to obtain the target compound 006 (22 mg), LC-MS (ESI-MS): 449[M+H]⁺.

### Example 7: N-(2,6-dimethyl-4-(7-(2,2,2-trifluoroethoxy)-1,3,4,5-tetrahydro-2H-benzo[c]azepin-2-yl)phenyl)-3,3-dimethylbutanamide (007)

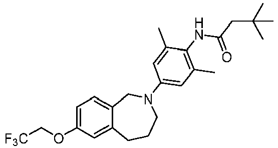

Step 1, 6-hydroxy-1-tetralone (2 g, 12.33 mmol) was dissolved in 15 mL of DMF, and the mixture was sequentially added with cesium carbonate (20.09 g, 61.66 mmol) and 2,2,2-trifluoroethyl p-toluenesulfonate (31.35 g, 123.31 mmol), and reacted overnight at 100°C. After the reaction was completed, the reaction mixture was cooled to room temperature, diluted with ethyl acetate, and filtered under reduced pressure. The organic phase was washed twice with saturated sodium bicarbonate, twice with saturated ammonium chloride, concentrated under reduced pressure, and subjected to silica gel column chromatography to obtain the product 6-(2,2,2-trifluoroethoxy)-1-tetralone (1.6 g) with a yield of 52%. LC-MS (ESI-MS): 245 [M+H]⁺.

The synthetic route and method of Example 1 were referenced in the subsequent steps, and 6-methoxy-1-tetralone was replaced by 6-(2,2,2-trifluoroethoxy)-1-tetralone to obtain the target compound 007 (50 mg), LC-MS (ESI-MS): 463 [M+H]⁺.

### Example 8: N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide (008)

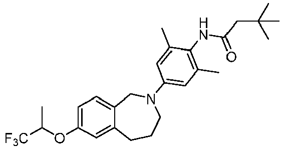

Step 1, 6-fluoro-1-tetralone (420 mg, 2.56 mmol) was dissolved in 10 mL of acetonitrile, and the mixture was sequentially added with cesium carbonate (1.67 g, 5.12 mmol) and trifluoroisopropanol (583.61 mg, 5.12 mmol), and refluxed overnight. After the reaction was completed, the reaction mixture was cooled to room temperature, diluted with ethyl acetate, filtered under reduced pressure, concentrated under reduced pressure, and subjected to silica gel column chromatography to obtain intermediate 008-1: 6-((1,1,1-trifluoropropan-2-yl)oxy)-1-tetralone (340 mg) with a yield of 51%. LC-MS (ESI-MS): 259[M+H]⁺.

Step 2, intermediate 008-1 (1.47 g, 5.67 mmol) was dissolved in 10 mL of concentrated hydrochloric acid, and the mixture was slowly added with sodium azide (737.86 mg, 11.35 mmol) at 0°C, gradually warmed to room temperature, and reacted for 6 hours. After the reaction was completed, the reaction mixture was slowly added with saturated potassium carbonate aqueous solution to adjust the pH of the system around 8, and extracted with ethyl acetate. The organic phases were combined, dried over anhydrous sodium sulfate, concentrated under reduced pressure, and subjected to silica gel column chromatography to obtain intermediate 008-2: 7-((1,1,1-trifluoropropan-2-yl)oxy)-2,3,4,5-tetrahydro-1H-benzo[c]azepin-1-one. LC-MS (ESI-MS): 274[M+H]⁺.

Step 3, intermediate 008-2 (274 mg, 1.0 mmol) was dissolved in 1 mL of tetrahydrofuran, and the mixture was slowly added dropwise with lithium aluminum hydride (2.6 mL, 5.23 mmol, 2M in THF) at 0°C, heated to reflux and reacted for 4 hours. After the reaction was completed, the reaction mixture was slowly added dropwise with water until the system stopped bubbling, and then concentrated under reduced pressure. The residue was dissolved with ethyl acetate and concentrated under reduced pressure to obtain intermediate 008-3: 7-((1,1,1-trifluoropropan-2-yl)oxy)-2,3,4,5-tetrahydro-1H-benzo[c]azepine, LC-MS (ESI-MS): 260[M+H]⁺.

Step 4, intermediate 008-3 (130 mg, 0.50 mmol) was dissolved in 2 mL of 1,4-dioxane, and the mixture was sequentially added with N-(4-bromo-2,6-dimethylphenyl)-3,3-dimethylbutanamide (252.38 mg, 0.84 mmol), Pd₂(dba)₃ (25.83 mg, 0.02 mmol), X-Phos (2-dicyclohexylphosphino-2',4',6'-triisopropylbiphenyl) (26.90 mg, 0.05 mmol), and sodium tert-butoxide (162.66 mg, 1.69 mmol), replaced with nitrogen, and reacted at 80°C for 2 hours. After the reaction was completed, the reaction mixture was cooled to room temperature and filtered under reduced pressure. The solid residue was washed with ethyl acetate and then washed three times with saturated ammonium chloride solution. The organic phase was concentrated and subjected to silica gel column chromatography to obtain the target compound 008 (90 mg). LC-MS (ESI-MS): 477[M+H]⁺. ¹H NMR (400 MHz, CDCl₃) δ 7.19 (d, *J* = 8.2 Hz, 1H), 6.73 (d, *J* = 2.6 Hz, 1H), 6.67 (dd, *J* = 8.2, 2.6 Hz, 1H), 6.47 (s, 2H), 6.41 (s, 1H), 4.61 - 4.53 (m, 1H), 4.49 (s, 2H), 3.72 (s, 2H), 2.96 - 2.88 (m, 2H), 2.23 (s, 2H), 2.15 (s, 6H), 1.90 - 1.81 (m, 2H), 1.46 (d, *J* = 6.4 Hz, 3H), 1.12 (s, 9H).

### Example 9: N-(4-(7-cyclopropoxy-1,3,4,5-tetrahydro-2H-benzo[c]azepin-2-yl)-2,6-dimethylphenyl)-3,3-dimethylbutanamide (009)

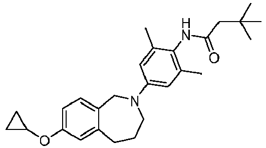

With reference to the synthetic route and method of Example 1, iodomethane was replaced by bromocyclopropane to obtain the target compound 009 (21 mg), LC-MS (ESI-MS): 421 [M+H]⁺.

### Example 10: N-(4-(7-cyclobutoxy-1,3,4,5-tetrahydro-2H-benzo[c]azepin-2-yl)-2,6-dimethylphenyl)-3,3-dimethylbutanamide (010)

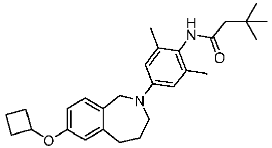

With reference to the synthetic route and method of Example 1, iodomethane was replaced by bromocyclobutane to obtain the target compound 010 (63 mg), LC-MS (ESI-MS): 435[M+H]⁺. ¹H NMR (400 MHz, CDCl₃) δ 7.13 (d, *J* = 8.2 Hz, 1H), 6.59 (d, *J* = 2.4 Hz, 1H), 6.52 (dd, *J* = 8.1, 2.6 Hz, 1H), 6.48 (d, *J* = 6.6 Hz, 2H), 6.40 (s, 1H), 4.57 (t, *J* = 7.2 Hz, 1H), 4.48 (d, *J* = 10.8 Hz, 2H), 3.70 (d, *J* = 4.8 Hz, 2H), 2.93 - 2.84 (m, 2H), 2.45 - 2.36 (m, 2H), 2.23 (s, 2H), 2.15 (s, 6H), 1.88 - 1.77 (m, 4H), 1.25 (s, 2H), 1.12 (s, 9H).

### Example 11: N-(4-(7-(cyclopentyloxy)-1,3,4,5-tetrahydro-2H-benzo[c]azepin-2-yl)-2,6-dimethylphenyl)-3,3-dimethylbutanamide (011)

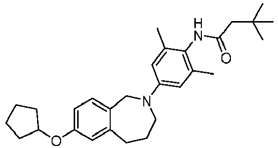

With reference to the synthetic route and method of Example 1, iodomethane was replaced by bromocyclopentane to obtain the target compound 011 (52 mg), LC-MS (ESI-MS): 449[M+H]⁺. ¹H NMR (400 MHz, CDCl₃) δ 7.14 (d, *J* = 8.2 Hz, 1H), 6.63 (d, *J* = 2.5 Hz, 1H), 6.58 (dd, *J* = 8.2, 2.6 Hz, 1H), 6.48 (s, 2H), 6.40 (brs, 1H), 4.71 - 4.65 (m, 1H), 4.47 (s, 2H), 3.75 - 3.67 (m, 2H), 2.93 - 2.86 (m, 2H), 2.23 (s, 2H), 2.15 (s, 6H), 1.89 - 1.81 (m, 6H), 1.62 - 1.54 (m, 4H), 1.12 (s, 9H).

### Example 12: N-(4-(7-(cyclohexyloxy)-1,3,4,5-tetrahydro-2H-benzo[c]azepin-2-yl)-2,6-dimethylphenyl)-3,3-dimethylbutanamide (012)

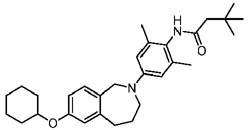

With reference to the synthetic route and method of Example 1, iodomethane was replaced by bromocyclohexane to obtain the target compound 012 (57 mg), LC-MS (ESI-MS): 463 [M+H]⁺.

### Example 13: N-(2,6-dimethyl-4-(7-(oxetan-3-yloxy)-1,3,4,5-tetrahydro-2H-benzo[c]azepin-2-yl)phenyl)-3,3-dimethylbutanamide (013)

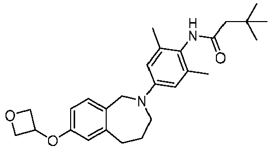

With reference to the synthetic route and method of Example 1, iodomethane was replaced by 3-bromo-oxetane to obtain the target compound 013 (22 mg), LC-MS (ESI-MS): 437[M+H]⁺.

### Example 14: N-(2,6-dimethyl-4-(7-(methylthio)-1,3,4,5-tetrahydro-2H-benzo[c]azepin-2-yl)phenyl)-3,3-dimethylbutanamide (014)

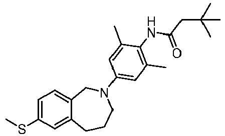

Step 1, 6-fluoro-1-tetralone (1.6 g) was dissolved in 15 mL of anhydrous DMSO in a reaction flask, and the mixture was added with sodium thiomethoxide (1.1 g), heated to 50°C, stirred, and reacted for about 3 hours. The reaction mixture was added with water to quench the reaction and added with dichloromethane and water to extract. The separated dichloromethane phase was dried over anhydrous sodium sulfate and concentrated, and then purified by silica gel column chromatography to obtain 6-(methylthio)-1-tetralone (1.1 g), LC-MS (ESI-MS): 193 [M+H]⁺.

The synthetic route and method of Example 1 were referenced in the subsequent steps, and 6-methoxy-1-tetralone was replaced by 6-(methylthio)-1-tetralone to obtain the target compound 014 (78 mg), LC-MS (ESI-MS): 411[M+H]⁺. ¹H NMR (400 MHz, CDCl₃) δ 7.18 (d, *J* = 7.6 Hz, 1H), 7.00 (d, *J* = 8.2 Hz, 2H), 6.47 (s, 2H), 6.41 (s, 1H), 4.50 (s, 2H), 3.74 - 3.69 (m, 2H), 2.95 - 2.89 (m, 2H), 2.44 (s, 3H), 2.23 (s, 2H), 2.14 (s, 6H), 1.88 - 1.83 (m, 2H), 1.12 (s, 9H).

### Example 15: N-(2,6-dimethyl-4-(7-(methylsulfonyl)-1,3,4,5-tetrahydro-2H-benzo[c]azepin-2-yl)phenyl)-3,3-dimethylbutanamide (015)

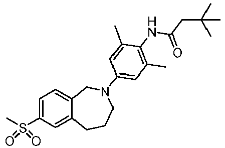

Compound 014 (30 mg) was dissolved in 3 mL of dichloromethane in a reaction flask, and mCPBA (35 mg) (3-chloroperoxybenzoic acid) was added thereto, and the reaction mixture was stirred at room temperature for about 1 hour, then quenched with sodium sulfite aqueous solution, and added with dichloromethane and water for extraction, and the separated dichloromethane phase was dried over anhydrous sodium sulfate and concentrated, and then purified by silica gel column chromatography to obtain compound 015 (26 mg), LC-MS (ESI -MS): 443 [M+H]⁺.

### Example 16: N-(4-(7-fluoro-5,5-dimethyl-1,3,4,5-tetrahydro-2H-benzo[c]azepin-2-yl)-2,6-dimethylphenyl)-3,3-dimethylbutanamide (016)

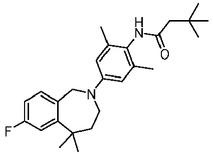

Step 1, 6-fluoro-4,4-dimethyl-1-tetralone (700 mg obtained by referring to the synthesis method of Adv. Synth. Catal. 2019, 361, 3223-3227) was dissolved in 7 mL of hydrochloric acid, and sodium azide (473.46 mg, 7.28 mmol) was slowly added thereto at 0°C. The reaction mixture was gradually warmed to room temperature and reacted for 4 hours. After the reaction was completed, the reaction mixture was slowly added with saturated potassium carbonate aqueous solution to adjust the pH of the system around 8, and extracted with ethyl acetate. The organic phases were combined, dried over anhydrous sodium sulfate, concentrated under reduced pressure, and subjected to silica gel column chromatography to obtain intermediate 016-1: 7-fluoro-5,5-dimethyl-2,3,4,5-tetrahydro-1H-benzo[c]azepin-1-one (360 mg) with a yield of 36%. LC-MS (ESI-MS): 208[M+H]⁺.

Step 2, intermediate 016-1 (170 mg, 0.82 mmol) was dissolved in 3 mL of tetrahydrofuran, and the mixture was slowly added dropwise with lithium aluminum hydride (1.6 mL, 3.28 mmol, 2M in THF) at 0°C, heated to reflux, and reacted for 4 hours. After the reaction was completed, the reaction mixture was slowly added dropwise with water until the system stopped bubbling, and then concentrated under reduced pressure. The residue was dissolved with ethyl acetate and concentrated under reduced pressure to obtain intermediate 016-2: 7-fluoro-5,5-dimethyl-2,3,4,5-tetrahydro-1H-benzo[c]azepine (150 mg) with a yield of 94%. LC-MS (ESI-MS): 194[M+H]⁺.

Step 3, N-(2,6-dimethyl-4-bromophenyl)-3,3-dimethylbutanamide (5 g, 16.77 mmol) was dissolved in 80 mL of 1,4-dioxane, and the mixture was sequentially added with bis(pinacolato)diboron (3.41 g, 13.41 mmol), potassium acetate (4.94 g, 50.30 mmol), and Pd(dppf)Cl₂ (614.62 mg, 0.83 mmol), replaced with nitrogen, and reacted at 90°C for 3 hours. After the reaction was completed, the reaction mixture was diluted with ethyl acetate and filtered. The organic phase was washed once with saturated sodium chloride aqueous solution, dried, concentrated under reduced pressure, and subjected to column chromatography to obtain intermediate 016-3: (3,5-dimethyl-4-(3,3-dimethylbutanamido))phenylboronic acid pinacol ester (4.30 g) with a yield of 74%. LC-MS (ESI-MS): 346[M+H]⁺.

Step 4, intermediate 016-3 (3 g, 8.69 mmol) was dissolved in 30 mL of tetrahydrofuran, and the mixture was sequentially added with 10 mL of water and sodium periodate (11.15 g, 52.13 mmol), and reacted overnight at room temperature. After the reaction was completed, the reaction mixture was added with water until a solid precipitated, filtered, and dried to obtain intermediate 016-4: (3,5-dimethyl-4-(3,3-dimethylbutanamido))phenylboronic acid (2.0 g) with a yield of 87%. LC-MS (ESI-MS): 264[M+H]⁺.

Step 5, intermediate 016-2 (100 mg, 0.51 mmol) was dissolved in 5 mL of acetonitrile, and the mixture was sequentially added with intermediate 016-4 (204.24 mg, 0.77 mmol), boric acid (63.99 mg, 1.03 mmol), N,N-diisopropylethylamine (133.75 mg, 1.03 mmol), and copper acetate (93.98 mg, 0.51 mmol), and reacted at 70°C for 3 hours under oxygen atmosphere. After the reaction was completed, the reaction mixture was cooled to room temperature, diluted with ethyl acetate, and filtered under reduced pressure. The organic phase was washed twice with saturated ammonium chloride solution, dried, concentrated under reduced pressure, and subjected to column chromatography to obtain the target compound 016 (70 mg) with a yield of 32%. LC-MS (ESI-MS): 411[M+H]⁺, ¹H NMR (400 MHz, CDCl₃) δ 7.22 - 7.17 (m, 1H), 7.07 (dd, *J* = 11.7, 2.6 Hz, 1H), 6.82 (td, *J* = 8.0, 2.6 Hz, 1H), 6.43 - 6.38 (m, 3H), 4.56 (s, 2H), 3.61 - 3.55 (m, 2H), 2.23 (s, 2H), 2.14 (s, 6H), 2.05 - 1.98 (m, 2H), 1.33 (d, *J* = 4.0 Hz, 6H), 1.12 (s, 9H).

### Example 17: N-(4-(5,5-dimethyl-1,3,4,5-tetrahydro-2H-benzo[c]azepin-2-yl)-2,6-dimethylphenyl)-3,3-dimethylbutanamide (017)

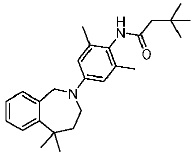

With reference to the synthetic route and method of Example 16, 6-fluoro-4,4-dimethyl-1-tetralone was replaced by 4,4-dimethyl-1-tetralone (obtained by referring to the synthesis method of Adv. Synth. Catal. 2019, 361, 3223-3227) to obtain the target compound 017 (52 mg), LC-MS (ESI-MS): 393[M+H]⁺, ¹H NMR (400 MHz, CDCl₃) δ 7.39 (d, *J* = 7.0 Hz, 1H), 7.24 - 7.08 (m, 3H), 6.42 (d, *J* = 5.9 Hz, 3H), 4.59 (s, 2H), 3.60 - 3.54 (m, 2H), 2.23 (s, 2H), 2.14 (s, 6H), 2.05 (t, *J* = 5.9 Hz, 2H), 1.33 (s, 6H), 1.12 (s, 9H).

### Example 18: N-(4-(2-chloro-8,8-dimethyl-4,6,7,8-tetrahydro-SH-thieno[3,2-c]azepin-5-yl)-2,6-dimethylphenyl)-3,3-dimethylbutanamide (018)

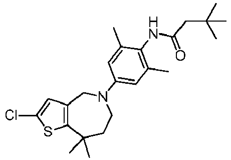

With reference to the synthetic route and method of Example 16, 6-fluoro-4,4-dimethyl-1-tetralone was replaced by 2-chloro-7,7-dimethyl-6,7-dihydrobenzo[b]thiophen-4(5H)-one (obtained by referring to the synthesis method of Adv. Synth. Catal. 2019, 361, 3223-3227) to obtain the target compound 018 (28 mg), LC-MS (ESI-MS): 433[M+H]⁺, ¹H NMR (400 MHz, CDCl₃) δ 6.71 (s, 1H), 6.45 - 6.40 (m, 3H), 4.37 (s, 2H), 3.76 - 3.70 (m, 2H), 2.25 (s, 2H), 2.16 (s, 6H), 1.90 - 1.84 (m, 2H), 1.33 (s, 6H), 1.13 (s, 9H).

### Example 19: N-(4-(8,8-dimethyl-4,6,7,8-tetrahydro-5H-thieno[3,2-c]azepin-5-yl)-2,6-dimethylphenyl)-3,3-dimethylbutanamide (019)

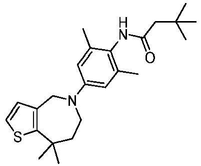

With reference to the synthetic route and method of Example 16, 6-fluoro-4,4-dimethyl-1-tetralone was replaced by 7,7-dimethyl-6,7-dihydrobenzo[b]thiophen-4(5H)-one (obtained by referring to the synthesis method of Adv. Synth. Catal. 2019, 361, 3223-3227) to obtain the target compound 019 (41 mg), LC-MS (ESI-MS): 399[M+H]⁺, ¹H NMR (400 MHz, CDCl₃) δ 6.97 (d, *J* = 5.1 Hz, 1H), 6.90 (d, *J* = 5.1 Hz, 1H), 6.47 (s, 2H), 6.42 (brs, 1H), 4.48 (s, 2H), 3.81 - 3.76 (m, 2H), 2.24 (s, 2H), 2.15 (s, 6H), 1.91 - 1.86 (m, 2H), 1.38 (s, 6H), 1.12 (s, 9H).

### Example 20: N-(4-(2-chloro-4,4-dimethyl-4,5,6,8-tetrahydro-7H-thieno[2,3-c]azepin-7-yl)-2,6-dimethylphenyl)-3,3-dimethylbutanamide (020)

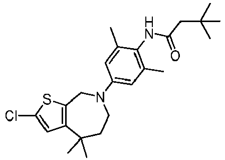

With reference to the synthetic route and method of Example 16, 6-fluoro-4,4-dimethyl-1-tetralone was replaced by 2-chloro-4,4-dimethyl-5,6-dihydrobenzo[b]thiophen-7(4H)-one (obtained by referring to the synthesis method of Adv. Synth. Catal. 2019, 361, 3223-3227) to obtain the target compound 020 (29 mg), LC-MS (ESI-MS): 433[M+H]⁺, ¹H NMR (400 MHz, CDCl₃) δ 6.73 (s, 1H), 6.49 - 6.44 (m, 1H), 4.44 (s, 2H), 3.72 - 3.68 (m, 2H), 2.25 (s, 2H), 2.17 (s, 6H), 1.89 - 1.83 (m, 2H), 1.24 (s, 6H), 1.13 (s, 9H).

### Example 21: N-(4-(4,4-dimethyl-4,5,6,8-tetrahydro-7H-thieno[2,3-c]azepin-7-yl)-2,6-dimethylphenyl)-3,3-dimethylbutanamide (021)

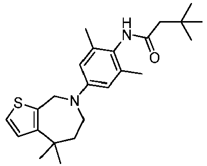

With reference to the synthetic route and method of Example 16, 6-fluoro-4,4-dimethyl-1-tetralone was replaced by 4,4-dimethyl-5,6-dihydrobenzo[b]thiophen-7(4H)-one (obtained by referring to the synthesis method of Adv. Synth. Catal. 2019, 361, 3223-3227) to obtain the target compound 021 (18 mg), LC-MS (ESI-MS): 399[M+H]⁺, ¹H NMR (400 MHz, CDCl₃) δ 6.98 (d, *J* = 5.2 Hz, 1H), 6.91 (d, *J* = 5.2 Hz, 1H), 6.53 (s, 2H), 6.42 (brs, 1H), 4.57 (s, 2H), 3.77 - 3.72 (m, 2H), 2.24 (s, 2H), 2.16 (s, 6H), 1.92 - 1.88 (m, 2H), 1.28 (s, 6H), 1.12 (s, 9H).

### Example 22: N-(4-(7-fluoro-3,4-dihydrospiro[benzo[c]azepine-5,1'-cyclopropan]-2(1H)-yl)-2,6-dimethylphenyl)-3,3-dimethylbutanamide (022)

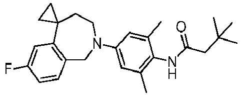

With reference to the synthetic route and method of Example 16, 6-fluoro-4,4-dimethyl-1-tetralone was replaced by 7'-fluoro-2',3'-dihydro-4'H-spiro[cyclopropane-1,1'-naphthalen]-4'-one (obtained by referring to the synthesis method of Adv. Synth. Catal. 2019, 361, 3223-3227) to obtain the target compound 022 (33 mg), LC-MS (ESI-MS): 409[M+H]⁺, ¹H NMR (400 MHz, DMSO) δ 8.72 (s, 1H), 7.48 (dd, *J* = 8.1, 6.2 Hz, 1H), 6.93 (ddd, *J* = 11.0, 9.5, 2.6 Hz, 2H), 6.54 (s, 2H), 4.67 (s, 2H), 3.78 (s, 2H), 2.15 (s, 2H), 2.05 (s, 6H), 1.65 - 1.46 (m, 2H), 1.05 (s, 9H), 0.96 - 0.83 (m, 4H).

### Example 23: N-(4-(8-fluoro-1,2,3,5-tetrahydro-4H-benzo[e][1,4]diazepin-4-yl)-2,6-dimethylphenyl)-3,3-dimethylbutanamide (023)

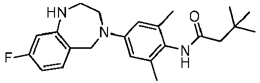

With reference to the synthetic route and method of Example 16, 6-fluoro-4,4-dimethyl-1-tetralone was replaced by 7-fluoro-2,3-dihydroquinolin-4(1H)-one (obtained by referring to the synthesis method of Bioorganic Chemistry, 2020, 99, 103800) to obtain the target compound 023 (44 mg), LC-MS (ESI-MS): 384[M+H]⁺, ¹H NMR (400 MHz, CDCl₃) δ 7.16 (dd, *J* = 8.2, 6.5 Hz, 1H), 6.53 - 6.49 (m, 2H), 6.48 (s, 2H), 6.39 (dd, *J* = 10.3, 2.4 Hz, 1H), 4.46 (s, 2H), 3.71 - 3.66 (m, 2H), 3.26 - 3.21 (m, 2H), 2.22 (s, 2H), 2.13 (s, 6H), 1.11 (s, 9H).

### Example 24: N-(4-(8-fluoro-1-methyl-1,2,3,5-tetrahydro-4H-benzo[e][1,4]diazepin-4-yl)-2,6-dimethylphenyl)-3,3-dimethylbutanamide (024)

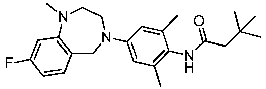

With reference to the synthetic route and method of Example 16, 6-fluoro-4,4-dimethyl-1-tetralone was replaced by 7-fluoro-1-methyl-2,3-dihydroquinolin-4(1H)-one to obtain the target compound 024 (52 mg), LC-MS (ESI-MS): 398[M+H]⁺. ¹H NMR (400 MHz, CDCl₃) δ 7.16 (dd, *J* = 8.9, 6.8 Hz, 1H), 6.56 (s, 1H), 6.55 - 6.50 (m, 3H), 6.44 (brs, 1H), 4.44 (s, 2H), 3.66 - 3.61 (m, 2H), 3.15 (dd, *J* = 5.9, 3.7 Hz, 2H), 2.88 (s, 3H), 2.24 (s, 2H), 2.16 (s, 6H), 1.12 (s, 9H).

### Example 25: N-(4-(8-fluoro-2,3-dihydrobenzo[f][1,4]thiazepin-4(5H)-yl)-2,6-dimethylphenyl)-3,3-dimethylbutanamide (025)

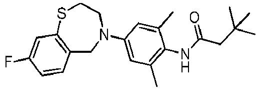

With reference to the synthetic route and method of Example 16, 6-fluoro-4,4-dimethyl-1-tetralone was replaced by 7-fluorothiochroman-4-one to obtain the target compound 025 (47 mg), LC-MS (ESI-MS): 401[M+H]⁺. ¹H NMR (400 MHz, CDCl₃) δ 7.33 (dd, *J* = 8.2, 5.9 Hz, 1H), 7.23 (dd, *J* = 8.8, 2.5 Hz, 1H), 6.86 (td, *J* = 8.3, 2.5 Hz, 1H), 6.49 - 6.41 (m, 3H), 4.71 (s, 2H), 4.03 - 3.96 (m, 2H), 2.97 - 2.91 (m, 2H), 2.23 (s, 2H), 2.15 (s, 6H), 1.11 (s, 9H).

### Example 26: N-(4-(8-fluoro-1,1-dioxido-2,3-dihydrobenzo[f][1,4]thiazepin-4(5H)-yl)-2,6-dimethylphenyl)-3,3-dimethylbutanamide (026)

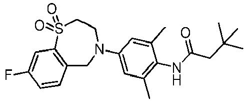

Compound 025 (25 mg) was dissolved in 2 mL of dichloromethane in a reaction flask, and mCPBA (30 mg) was added thereto, and the reaction mixture was stirred at room temperature for about 1 hour, then quenched with sodium sulfite aqueous solution, and added with dichloromethane and water for extraction. The separated dichloromethane phase was dried over anhydrous sodium sulfate and concentrated, and then purified by silica gel column chromatography to obtain the target compound 026 (21 mg), LC-MS (ESI -MS): 433 [M+H]⁺.

### Example 27: N-(4-(7-fluoro-1,3-dihydro-2H-benzo[c]azepin-2-yl)-2,6-dimethylphenyl)-3,3-dimethylbutanamide (027)

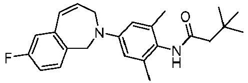

With reference to the synthetic route and method of Example 16, 6-fluoro-4,4-dimethyl-1-tetralone was replaced by 6-fluoronaphthalen-1(2H)-one to obtain the target compound 027 (37 mg), LC-MS (ESI-MS): 381[M+H]⁺. ¹H NMR (400 MHz, CDCl₃) δ 7.25 - 7.20 (m, 1H), 6.83 (t, *J* = 8.0 Hz, 2H), 6.47 (s, 2H), 6.42 - 6.33 (m, 2H), 6.03 - 5.96 (m, 1H), 4.49 (s, 2H), 4.27 (s, 2H), 2.23 (s, 2H), 2.13 (s, 6H), 1.12 (s, 9H).

### Example 28: N-(4-(7-fluoro-5-methyl-1,3-dihydro-2H-benzo[c]azepin-2-yl)-2,6-dimethylphenyl)-3,3-dimethylbutanamide (028)

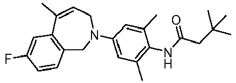

With reference to the synthetic route and method of Example 16, 6-fluoro-4,4-dimethyl-1-tetralone was replaced by 6-fluoro-4-methylnaphthalen-1(2H)-one to obtain the target compound 028 (23 mg), LC-MS (ESI-MS): 395[M+H]⁺. ¹H NMR (400 MHz, CDCl₃) δ 7.34 - 7.31 (m, 1H), 7.11 (dd, *J* = 10.2, 2.6 Hz, 1H), 6.97 (td, *J* = 8.3, 2.7 Hz, 1H), 6.60 (s, 2H), 6.53 (s, 1H), 6.07 (t, *J* = 5.8 Hz, 1H), 4.18 (s, 2H), 3.66 (d, *J* = 6.3 Hz, 2H), 2.31 (s, 2H), 2.24 (s, 6H), 2.20 (s, 3H), 1.19 (s, 9H).

### Example 29: N-(3-fluoro-4-(7-fluoro-1,3,4,5-tetrahydro-2H-benzo[c]azepin-2-yl)-2-methylphenyl)-3,3-dimethylbutanamide (029)

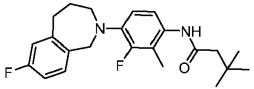

Step 1, N-(2-methyl-3-fluoro-4-bromophenyl)-3,3-dimethylbutanamide (5 g, 16.77 mmol) was dissolved in 80 mL of 1,4-dioxane, and the mixture was sequentially added with bis(pinacolato)diboron (3.41 g, 13.41 mmol), potassium acetate (4.94 g, 50.30 mmol), and Pd(dppf)Cl₂ (614.62 mg, 0.83 mmol), replaced with nitrogen, and reacted at 90°C for 3 hours. After the reaction was completed, the reaction mixture was diluted with ethyl acetate, and filtered. The organic phase was washed once with saturated sodium chloride aqueous solution, dried, concentrated under reduced pressure, and subjected to column chromatography to obtain oily (3-methyl-2-fluoro-4-(3,3-dimethylbutanamido))phenylboronic acid pinacol ester (4 g) with a yield of 69%. LC-MS (ESI-MS): 350[M+H]⁺.

Step 2, 7-fluoro-2,3,4,5-tetrahydro-1H-2-benzoazepine (90 mg, 0.54 mmol) was dissolved in 5 mL of acetonitrile, and the mixture was sequentially added with (3-methyl-2-fluoro-4-(3,3-dimethylbutanamido))phenylboronic acid pinacol ester (190 mg, 0.54 mmol), boric acid (70 mg, 1.09 mmol), and copper acetate (100 mg, 0.54 mmol), and reacted under oxygen atmosphere for 6 hours at 80°C. After the reaction was completed, the reaction mixture was cooled to room temperature, diluted with ethyl acetate, and filtered under reduced pressure. The organic phase was washed twice with saturated ammonium chloride solution, dried, concentrated under reduced pressure, and subjected to column chromatography to obtain the target compound 029 (36 mg) with a yield of 17%. LC-MS (ESI-MS):387[M+H]⁺. ¹H NMR (400 MHz, CDCl₃) δ 7.23 (d, *J* = 8.8 Hz, 1H), 7.17 - 7.11 (m, 1H), 6.85 (dd, *J* = 9.6, 2.3 Hz, 1H), 6.82 - 6.70 (m, 3H), 4.35 (s, 2H), 3.60 - 3.55 (m, 2H), 3.00 - 2.94 (m, 2H), 2.23 (s, 2H), 2.15 (d, *J* = 2.2 Hz, 3H), 1.98 - 1.91 (m, 2H), 1.11 (s, 9H).

### Example 30: N-(4-(7-fluoro-1,3,4,5-tetrahydro-2H-benzo[c]azepin-2-yl)-2,3,6-trimethylphenyl)-3,3-dimethylbutanamide (030)

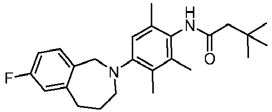

7-Fluoro-2,3,4,5-tetrahydro-1H-benzoazepine (100 mg, 0.60 mmol) was dissolved in 5 mL of acetonitrile, and the mixture was sequentially added with (2,3,5-trimethyl-4-(3,3-dimethylbutanamido))phenylboronic acid pinacol ester (260 mg, 0.72 mmol), boric acid (75 mg, 1.21 mmol), copper acetate (109 mg, 0.60 mmol), and pyridine (50 µL, 1.21 mmol), and reacted under oxygen atmosphere for 6 hours at 80°C. After the reaction was completed, the reaction mixture was cooled to room temperature, diluted with ethyl acetate, and filtered under reduced pressure. The organic phase was washed twice with saturated ammonium chloride solution, dried, concentrated under reduced pressure, and subjected to column chromatography to obtain the target compound 030 (38 mg) with a yield of 16%. LC-MS (ESI-MS):397[M+H]⁺. ¹H NMR (400 MHz, CDCl₃) δ 7.02 (dd, *J* = 8.2, 5.8 Hz, 1H), 6.89 - 6.85 (m, 2H), 6.79 (td, *J* = 8.4, 2.6 Hz, 1H), 6.58 (brs, 1H), 4.01 (s, 2H), 3.26 - 3.21 (m, 2H), 2.96 - 2.90 (m, 2H), 2.30 (s, 2H), 2.22 (s, 3H), 2.15 (s, 6H), 1.94 - 1.87 (m, 2H), 1.16 (s, 9H).

### Example 31: N-(3-fluoro-4-(7-fluoro-1,3,4,5-tetrahydro-2H-benzo[c]azepin-2-yl)-2,6-dimethylphenyl)-3,3-dimethylbutanamide (031)

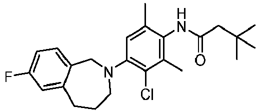

7-Fluoro-2,3,4,5-tetrahydro-1H-benzoazepine (100 mg, 0.60 mmol) was dissolved in 5 mL of acetonitrile, and the mixture was sequentially added with (2-chloro-3,5-dimethyl-4-(3,3-dimethylbutanamido))phenylboronic acid pinacol ester (282 mg, 0.66 mmol) (could be synthesized by a method similar to Example 16), boric acid (75 mg, 1.21 mmol), copper acetate (110 mg, 0.60 mmol), and pyridine (50 µL, 1.21 mmol), and reacted under oxygen atmosphere for 6 hours at 80°C. After the reaction was completed, the reaction mixture was cooled to room temperature, diluted with ethyl acetate, and filtered under reduced pressure. The organic phase was washed twice with saturated ammonium chloride solution, dried, concentrated under reduced pressure, and subjected to column chromatography to obtain the target compound 031 (52 mg) with a yield of 20%. LC-MS (ESI-MS):417[M+H]⁺. ¹H NMR (400 MHz, CDCl₃) δ 7.14 (dd, *J* = 8.3, 5.9 Hz, 1H), 6.89 - 6.85 (m, 2H), 6.80 (td, *J* = 8.5, 2.7 Hz, 1H), 6.63 (brs, 1H), 4.13 (s, 2H), 3.41 - 3.36 (m, 2H), 2.98 - 2.92 (m, 2H), 2.32 - 2.27 (m, 2H), 2.30 (s, 3H), 2.19 (s, 3H), 1.99 - 1.92 (m, 2H), 1.15 (s, 9H).

### Example 32: (R)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide (032)

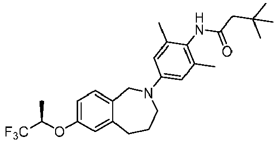

With reference to the synthetic route and method of Example 8, trifluoroisopropanol was replaced by (*R*)-1,1,1-trifluoropropan-2-ol to obtain the target compound 032 (50 mg). LC-MS (ESI-MS): 477[M+H]⁺. ¹H NMR (400 MHz, Chloroform-*d*) δ 7.19 (d, *J* = 8.2 Hz, 1H), 6.72 - 6.73 (m, 1H), 6.65 - 6.68 (m, 1H), 6.46 - 6.48 (m, 2H), 6.41 (brs, 1H), 4.53 - 4.59 (m, 1H), 4.49 (s, 2H), 3.71 - 3.73 (m, 2H), 2.89 - 2.92 (m, 2H), 2.22 (s, 2H), 2.14 (s, 6H), 1.83 - 1.86 (m, 2H), 1.46 (d, *J* = 6.5 Hz, 3H), 1.12 (s, 9H).

### Example 33: (S)-N-(2,6-dimethyl-4-(7-((1,1,1-trifluoropropan-2-yl)oxy)-1,3,4,5-tetrahydro-2H-benzo[c]azepin-2-yl)phenyl)-3,3-dimethylbutanamide (033)

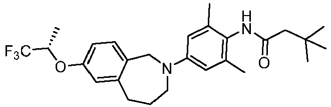

With reference to the synthetic route and method of Example 8, trifluoroisopropanol was replaced by (*S*)-1,1,1-trifluoropropan-2-ol to obtain the target compound 033 (63 mg). LC-MS (ESI-MS): 477[M+H]⁺. ¹H NMR (400 MHz, Chloroform-*d*) δ 7.19 (d, *J* = 8.2 Hz, 1H), 6.72 - 6.73 (m, 1H), 6.65 - 6.68 (m, 1H), 6.46 - 6.49 (m, 2H), 6.42 (brs, 1H), 4.52 - 4.60 (m, 1H), 4.49 (s, 2H), 3.71 - 3.73 (m, 2H), 2.90 - 2.93 (m, 2H), 2.22 (s, 2H), 2.14 (s, 6H), 1.82 - 1.88 (m, 2H), 1.45 - 1.46 (m, 3H), 1.12 (s, 9H).

### Example 34: N-(4-(8-fluoro-7-((1,1,1-trifluoropropan-2-yl)oxy)-1,3,4,5-tetrahydro-2H-benzo[c]azepin-2-yl)-2,6-dimethylphenyl)-3,3-dimethylbutanamide (034)

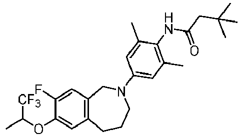

Step 1, 6,7-difluoro-1-tetralone (2.0 g, 10.98 mmol) was dissolved in 30 mL of acetonitrile, and the system in a sealed tube was sequentially added with trifluoroisopropanol (2.5 mL, 27.45 mmol) and cesium carbonate (10.7 g, 32.94 mmol), and the reaction mixture was reacted at 80°C for 4 hours. After the reaction was completed, the reaction mixture was concentrated to remove acetonitrile, added with ethyl acetate and water to dissolve, and extracted three times with ethyl acetate. The organic phases were combined, dried over anhydrous sodium sulfate, and concentrated under reduced pressure to obtain intermediate 034-1: 7-fluoro-6-((1,1,1-trifluoropropan-2-yl)oxy)-1-tetralone (3.12 g) with a yield of 94%. The intermediate 034-1 was used directly in the next step. ESI-MS (M+H)⁺ = 277. ¹H-NMR (400 MHz, CCl3D) δ 7.76 (d, J = 11.6 Hz, 1H), 6.89 (d, J = 7.5 Hz, 1H), 4.66 - 4.74 (m, 1H), 2.88 - 2.91 (m, 2H), 2.58 - 2.61 (m, 2H), 2.09 - 2.15 (m, 2H), 1.57 (dd, J = 6.5, 0.9 Hz, 3H).

Step 2, intermediate 034-1 (3.12 g, 11.30 mmol) was dissolved in 40mL of concentrated hydrochloric acid, and the mixture was slowly added with sodium azide (1.47 g, 22.60 mmol) in an ice bath, and reacted at room temperature for 48 hours. After the reaction was completed, the reaction mixture was slowly added with ice water and sodium carbonate aqueous solution at a low temperature to adjust the pH of the system to alkalinity, and extracted three times with ethyl acetate. The organic phases were combined, dried over anhydrous sodium sulfate, concentrated under reduced pressure, and subjected to column chromatography (PE/EA for elution) to obtain intermediate 034-2: 8-fluoro-7-((1,1,1-trifluoropropan-2-yl)oxy)-2,3,4,5-tetrahydro-1H-benzo[c]azepin-1-one (750 mg) with a yield of 23%. ESI-MS (M+H)⁺ = 292.

Step 3, intermediate 034-2 (400 mg, 1.44 mmol) was dissolved in 100 mL of tetrahydrofuran, and the mixture was added with lithium aluminum hydride (1 mmol/mL, 13 mL), and reacted at 60°C for 2 hours. After the reaction was completed, the reaction mixture was added with water to quench until the system stopped bubbling, and then concentrated under reduced pressure to remove water. The mixture was dissolved with ethyl acetate (1 g product per 1 L EA), filtered under reduced pressure, and dried over anhydrous sodium sulfate. The filtrate was concentrated under reduced pressure to obtain intermediate 034-3: 8-fluoro-7-((1,1,1-trifluoropropan-2-yl)oxy)-2,3,4,5-tetrahydro-1H-benzo[c]azepine (350 mg) with a yield of 92%. ESI-MS (M+H)⁺ = 278.

Step 4, intermediate 034-3 (230 mg, 0.83 mmol) was dissolved in 10 mL of 1,4-dioxane, and the system was sequentially added with N-(4-bromo-2,6-dimethylphenyl)-3,3-dimethylbutanamide (370 mg, 1.24 mmol), sodium tert-butoxide (244 mg, 2.49 mmol), cesium carbonate (811 mg, 2.49 mmol), palladium acetate (19 mg, 0.08 mmol), Pd₂(dba)₃ (76 mg, 0.08 mmol), and X-Phos (2-dicyclohexylphosphino-2',4',6'-triisopropylbiphenyl) (79 mg, 0.17 mmol), replaced with nitrogen, and the reaction mixture was reacted at 105°C for 4 hours. After the reaction was completed, the reaction mixture was concentrated under reduced pressure to remove dioxane, added with dichloromethane and water to dissolve the mixture, and extracted three times with dichloromethane. The organic phases were combined, dried over anhydrous sodium sulfate, concentrated under reduced pressure, and subjected to column chromatography (PE/EA for elution) to obtain the target compound 034 (170 mg) with a yield of 41%. ESI-MS (M+H)⁺ = 495. ¹H-NMR (400 MHz, CCl₃D) δ 7.02 (d, *J* = 11.2 Hz, 1H), 6.82 (d, *J* = 8.1 Hz, 1H), 6.43 - 6.47 (m, 3H), 4.45 - 4.49 (m, 3H), 3.67 - 3.75 (m, 2H), 2.87 - 2.90 (m, 2H), 2.23 (s, 2H), 2.16 (s, 6H), 1.82 - 1.85 (m, 2H), 1.50 (d, *J* = 6.5 Hz, 3H), 1.12 (s, 9H).

### Example 35: N-(4-(8-fluoro-7-isopropoxy-1,3,4,5-tetrahydro-2H-benzo[c]azepin-2-yl)-2,6-dimethylphenyl)-3,3-dimethylbutanamide (035)

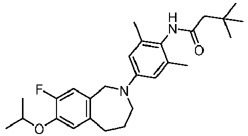

Step 1, with reference to the synthetic route and method of steps 1, 2, and 3 of Example 34, trifluoroisopropanol was replaced by isopropanol to obtain 8-fluoro-7-isopropoxy-2,3,4,5-tetrahydro-1H-benzo[c]azepine, ESI-MS (M+H)⁺ = 224.

Step 2, 8-fluoro-7-isopropoxy-2,3,4,5-tetrahydro-1H-benzo[c]azepine (540 mg, 2.42 mmol) was dissolved in 15 mL of acetonitrile, and the system was sequentially added with (4-(3,3-dimethylbutanamido)-3,5-dimethylphenyl)boronic acid (1.3 g, 4.84 mmol), copper acetate (440 mg, 2.42 mmol), boric acid (300 mg, 4.84 mmol), and DIPEA (940 mg, 7.26 mmol), replaced with oxygen, and the reaction mixture was reacted at 60°C for 2 hours. After the reaction was completed, the reaction mixture was added with saturated ammonium chloride aqueous solution to wash and extracted three times with dichloromethane. The organic phases were combined, dried over anhydrous sodium sulfate, concentrated under reduced pressure, and subjected to column chromatography (PE/EA for elution) to obtain the target compound 035 (150 mg) with a yield of 14%. ESI-MS (M+H)⁺ = 441. ¹H-NMR (400 MHz, CCl₃D) δ 6.97 - 7.00 (m, 1H), 6.71 - 6.74 (m, 1H), 6.42 - 6.48 (m, 3H), 4.40 - 4.45 (m, 3H), 3.67 - 3.73 (m, 2H), 2.85 - 2.89 (m, 2H), 2.21 (s, 2H), 2.14 (s, 6H), 1.81 - 1.85 (m, 2H), 1.32 (d, *J* = 6.1 Hz, 6H), 1.12 (s, 9H).

### Example 36. (S)-N-(4-(8-fluoro-7-((1,1,1-trifluoropropan-2-yl)oxy)-1,3,4,5-tetrahydro-2H-benzo[c]azepin-2-yl)-2,6-dimethylphenyl)-3,3-dimethylbutanamide (036)

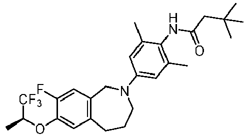

With reference to the synthetic route and method of Example 34, trifluoroisopropanol in step 1 was replaced by (S)-1,1,1-trifluoropropan-2-ol to obtain the target compound 036 (58 mg). LC-MS (ESI-MS): 495[M+H]⁺.

### Example 37. (R)-N-(4-(8-fluoro-7-((1,1,1-trifluoropropan-2-yl)oxy)-1,3,4,5-tetrahydro-2H-benzo[c]azepin-2-yl)-2,6-dimethylphenyl)-3,3-dimethylbutanamide (037)

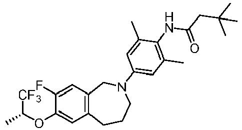

With reference to the synthetic route and method of Example 34, trifluoroisopropanol in step 1 was replaced by (R)-1,1,1-trifluoropropan-2-ol to obtain the target compound 037 (50 mg). LC-MS (ESI-MS): 495[M+H]⁺.

### Example 38. N-(4-(8-fluoro-7-methoxy-1,3,4,5-tetrahydro-2H-benzo[c]azepin-2-yl)-2,6-dimethylphenyl)-3,3-dimethylbutanamide (038)

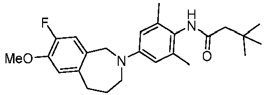

With reference to the synthetic route and method of Example 34, trifluoroisopropanol in step 1 was replaced by methanol to obtain the target compound 038 (47 mg). LC-MS (ESI-MS): 413[M+H]⁺.

### Example 39. N-(4-(7-(sec-butoxy)-8-fluoro-1,3,4,5-tetrahydro-2H-benzo[c]azepin-2-yl)-2,6-dimethylphenyl)-3,3-dimethylbutanamide (039)

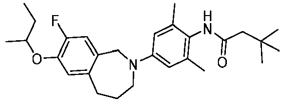

With reference to the synthetic route and method of Example 34, trifluoroisopropanol in step 1 was replaced by butan-2-ol to obtain the target compound 039 (62 mg). LC-MS (ESI-MS): 455[M+H]⁺.

### Example 40. (S)-N-(4-(7-(sec-butoxy)-8-fluoro-1,3,4,5-tetrahydro-2H-benzo[c]azepin-2-yl)-2,6-dimethylphenyl)-3,3-dimethylbutanamide (040)

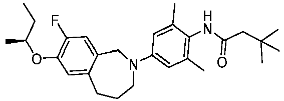

With reference to the synthetic route and method of Example 34, trifluoroisopropanol in step 1 was replaced by (S)-butan-2-ol to obtain the target compound 040 (40 mg). LC-MS (ESI-MS): 455[M+H]⁺.

### Example 41. (R)-N-(4-(7-(sec-butoxy)-8-fluoro-1,3,4,5-tetrahydro-2H-benzo[c]azepin-2-yl)-2,6-dimethylphenyl)-3,3-dimethylbutanamide (041)

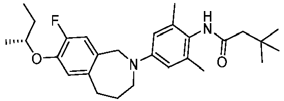

With reference to the synthetic route and method of Example 34, trifluoroisopropanol in step 1 was replaced by (R)-butan-2-ol to obtain the target compound 041 (46 mg). LC-MS (ESI-MS): 455[M+H]⁺.

### Example 42. N-(4-(7-cyclopropoxy-8-fluoro-1,3,4,5-tetrahydro-2H-benzo[c]azepin-2-yl)-2,6-dimethylphenyl)-3,3-dimethylbutanamide (042)

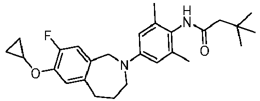

With reference to the synthetic route and method of Example 34, trifluoroisopropanol in step 1 was replaced by cyclopropanol to obtain the target compound 042 (35 mg). LC-MS (ESI-MS): 439[M+H]⁺.

### Example 43. N-(4-(7-cyclobutoxy-8-fluoro-1,3,4,5-tetrahydro-2H-benzo[c]azepin-2-yl)-2,6-dimethylphenyl)-3,3-dimethylbutanamide (043)

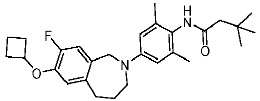

With reference to the synthetic route and method of Example 34, trifluoroisopropanol in step 1 was replaced by cyclobutanol to obtain the target compound 043 (34 mg). LC-MS (ESI-MS): 453 [M+H]⁺.

### Example 44. N-(4-(7-(cyclopentyloxy)-8-fluoro-1,3,4,5-tetrahydro-2H-benzo[c]azepin-2-yl)-2,6-dimethylphenyl)-3,3-dimethylbutanamide (044)

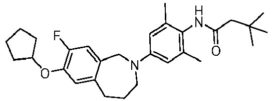

With reference to the synthetic route and method of Example 34, trifluoroisopropanol in step 1 was replaced by cyclopentanol to obtain the target compound 044 (41 mg). LC-MS (ESI-MS): 467[M+H]⁺.

### Example 45. N-(4-(7-(cyclohexyloxy)-8-fluoro-1,3,4,5-tetrahydro-2H-benzo[c]azepin-2-yl)-2,6-dimethylphenyl)-3,3-dimethylbutanamide (045)

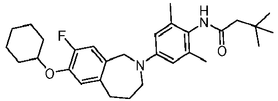

With reference to the synthetic route and method of Example 34, trifluoroisopropanol in step 1 was replaced by cyclohexanol to obtain the target compound 045 (48 mg). LC-MS (ESI-MS): 481[M+H]⁺.

### Example 46. N-(4-(7,8-difluoro-1,3-dihydro-2H-benzo[c]azepin-2-yl)-2,6-dimethylphenyl)-3,3-dimethylbutanamide (046)

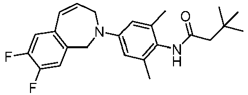

With reference to the synthetic route and method of steps 2, 3, and 4 of Example 34, 7-fluoro-6-((1,1,1-trifluoropropan-2-yl)oxy)-1-tetralone was replaced by 6,7-difluoronaphthalen-1(2H)-one to obtain the target compound 046 (38 mg). LC-MS (ESI-MS): 399[M+H]⁺.

### Example 47. N-(4-(7,8-difluoro-1,3,4,5-tetrahydro-2H-benzo[c]azepin-2-yl)-2,6-dimethylphenyl)-3,3-dimethylbutanamide (047)

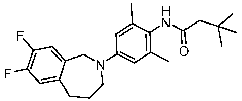

With reference to the synthetic route and method of steps 2, 3, and 4 of Example 34, 7-fluoro-6-((1,1,1-trifluoropropan-2-yl)oxy)-1-tetralone in step 2 was replaced by 6,7-difluoro-1-tetralone to obtain the target compound 047 (63 mg). LC-MS (ESI-MS): 401[M+H]⁺.

### Example 48. N-(3-chloro-4-(7,8-difluoro-1,3,4,5-tetrahydro-2H-benzo[c]azepin-2-yl)-2,6-dimethylphenyl)-3,3-dimethylbutanamide (048)

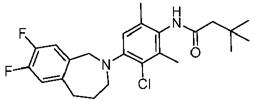

With reference to the synthetic route and method of Example 31, 7-fluoro-2,3,4,5-tetrahydro-1H-2-benzazepine was replaced by 7,8-difluoro-2,3,4,5-tetrahydro-1H-benzo[c]azepine to obtain the target compound 048 (37 mg). LC-MS (ESI-MS): 435[M+H]⁺.

### Example 49. N-(4-(7,8-difluoro-3,4-dihydrospiro[benzo[c]azepine-5,1'-cyclopropan]-2(1H)-yl)-2,6-dimethylphenyl)-3,3-dimethylbutanamide (049)

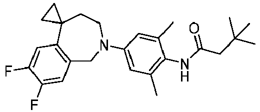

With reference to the synthetic route and method of steps 2, 3, and 4 of Example 34, 7-fluoro-6-((1,1,1-trifluoropropan-2-yl)oxy)-1-tetralone was replaced by 6',7'-difluoro-2',3'-dihydro-4'H-spiro[cyclopropane-1,1'-naphthalen]-4'-one to obtain the target compound 049 (38 mg). LC-MS (ESI-MS): 427[M+H]⁺.

### Example 50. (E)-N-(2,6-dimethyl-4-(7-((4,4,4-trifluorobut-2-en-1-yl)oxy)-1,3,4,5-tetrahydro-2H-benzo[c]azepin-2-yl)phenyl)-3,3-dimethylbutanamide (050)

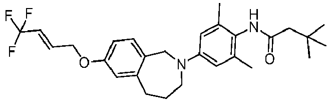

With reference to the synthetic route and method of Example 8, trifluoroisopropanol in step 1 was replaced by (E)-4,4,4-trifluorobut-2-en-1-ol to obtain the target compound 050 (50 mg). LC-MS (ESI-MS): 489[M+H]⁺.

### Example 51. N-(4-(7-((3-(difluoromethyl)bicyclo[1.1.1]pentan-1-yl)oxy)-1,3,4,5-tetrahydro-2H-benzo[c]azepin-2-yl)-2,6-dimethylphenyl)-3,3-dimethylbutanamide (051)

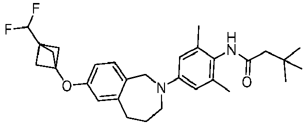

With reference to the synthetic route and method of Example 8, trifluoroisopropanol in step 1 was replaced by 3-(difluoromethyl)bicyclo[1.1.1]pentan-1-ol to obtain the target compound 051 (42 mg). LC-MS (ESI-MS): 497[M+H]⁺.

### Example 52. N-(2,6-dimethyl-4-(7-(2,2,2-trifluoro-1-methoxyethoxy)-1,3,4,5-tetrahydro-2H-benzo[c]azepin-2-yl)phenyl)-3,3-dimethylbutanamide (052)

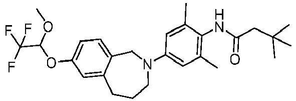

With reference to the synthetic route and method of Example 8, trifluoroisopropanol in step 1 was replaced by 2,2,2-trifluoro-1-methoxyethan-1-ol to obtain the target compound 052 (52 mg). LC-MS (ESI-MS): 493 [M+H]⁺.

### Example 53. N-(2,6-dimethyl-4-(7-(3,3,3-trifluoro-2-methylpropoxy)-1,3,4,5-tetrahydro-2H-benzo[c]azepin-2-yl)phenyl)-3,3-dimethylbutanamide (053)

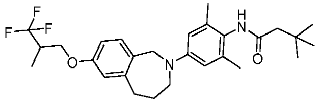

With reference to the synthetic route and method of Example 8, trifluoroisopropanol in step 1 was replaced by 3,3,3-trifluoro-2-methylpropan-1-ol to obtain the target compound 053 (46 mg). LC-MS (ESI-MS): 491[M+H]⁺.

### Example 54. N-(2,6-dimethyl-4-(7-((1-(trifluoromethyl)cyclopropyl)methoxy)-1,3,4,5-tetrahydro-2H-benzo[c]azepin-2-yl)phenyl)-3,3-dimethylbutanamide (054)

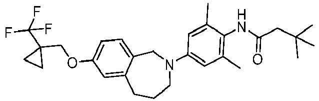

With reference to the synthetic route and method of Example 8, trifluoroisopropanol in step 1 was replaced by (1-(trifluoromethyl)cyclopropyl)methanol to obtain the target compound 054 (34 mg). LC-MS (ESI-MS): 503 [M+H]⁺.

### Example 55. N-(4-(7-((1,1,1,3,3,3-hexafluoropropan-2-yl)oxy)-1,3,4,5-tetrahydro-2H-benzo[c]azepin-2-yl)-2,6-dimethylphenyl)-3,3-dimethylbutanamide (055)

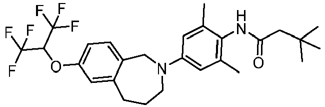

With reference to the synthetic route and method of Example 8, trifluoroisopropanol in step 1 was replaced by hexafluoroisopropanol to obtain the target compound 055 (49 mg). LC-MS (ESI-MS): 531 [M+H]⁺.

### Example 56. N-(2,6-dimethyl-4-(7-(2-(2,2,2-trifluoroethoxy)ethoxy)-1,3,4,5-tetrahydro-2H-benzo[c]azepin-2-yl)phenyl)-3,3-dimethylbutanamide (056)

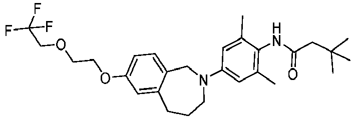

With reference to the synthetic route and method of Example 8, trifluoroisopropanol in step 1 was replaced by 2-(2,2,2-trifluoroethoxy)ethanol to obtain the target compound 056 (62 mg). LC-MS (ESI-MS): 507[M+H]⁺.

### Example 57. N-(2,6-dimethyl-4-(7-(2,2,3,3-tetrafluoropropoxy)-1,3,4,5-tetrahydro-2H-benzo[c]azepin-2-yl)phenyl)-3,3-dimethylbutanamide (057)

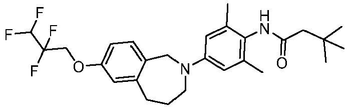

With reference to the synthetic route and method of Example 8, trifluoroisopropanol in step 1 was replaced by tetrafluoropropanol to obtain the target compound 057 (53 mg). LC-MS (ESI-MS): 495[M+H]⁺.

### Example 58. N-(2,6-dimethyl-4-(7-(3,3,3-trifluoropropoxy)-1,3,4,5-tetrahydro-2H-benzo[c]azepin-2-yl)phenyl)-3,3-dimethylbutanamide (058)

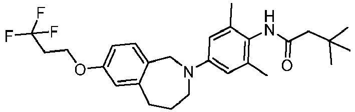

With reference to the synthetic route and method of Example 8, trifluoroisopropanol in step 1 was replaced by trifluoropropanol to obtain the target compound 058 (48 mg). LC-MS (ESI-MS): 477[M+H]⁺.

### Example 59. N-(2,6-dimethyl-4-(7-((4-(trifluoromethyl)cyclohexyl)oxy)-1,3,4,5-tetrahydro-2H-benzo[c]azepin-2-yl)phenyl)-3,3-dimethylbutanamide (059)

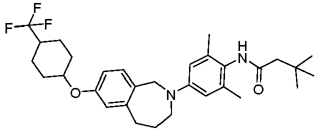

With reference to the synthetic route and method of Example 8, trifluoroisopropanol in step 1 was replaced by 4-(trifluoromethyl)cyclohexanol to obtain the target compound 059 (50 mg). LC-MS (ESI-MS): 531[M+H]⁺.

### Example 60. N-(2,6-dimethyl-4-(7-(4,4,4-trifluorobutoxy)-1,3,4,5-tetrahydro-2H-benzo[c]azepin-2-yl)phenyl)-3,3-dimethylbutanamide (060)

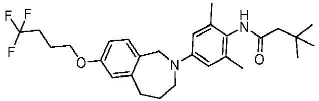

With reference to the synthetic route and method of Example 8, trifluoroisopropanol in step 1 was replaced by trifluorobutanol to obtain the target compound 060 (63 mg). LC-MS (ESI-MS): 491[M+H]⁺.

### Example 61. N-(2,6-dimethyl-4-(7-((4,4,5,5,5-pentafluoropentyl)oxy)-1,3,4,5-tetrahydro-2H-benzo[c]azepin-2-yl)phenyl)-3,3-dimethylbutanamide (061)

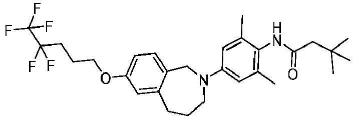

With reference to the synthetic route and method of Example 8, trifluoroisopropanol in step 1 was replaced by 4,4,5,5,5-pentafluoropentanol (49 mg). LC-MS (ESI-MS): 541 [M+H]⁺.

### Example 62. N-(2,6-dimethyl-4-(7-(2,2,4,4,4-pentafluorobutoxy)-1,3,4,5-tetrahydro-2H-benzo[c]azepin-2-yl)phenyl)-3,3-dimethylbutanamide (062)

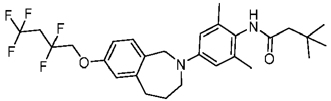

With reference to the synthetic route and method of Example 8, trifluoroisopropanol in step 1 was replaced by 2,2,4,4,4-pentafluorobutanol (57 mg). LC-MS (ESI-MS): 527[M+H]⁺.

### Example 63. N-(4-(7-(4-fluorobutoxy)-1,3,4,5-tetrahydro-2H-benzo[c]azepin-2-yl)-2,6-dimethylphenyl)-3,3-dimethylbutanamide (063)

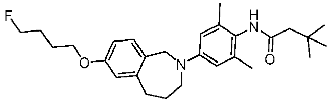

With reference to the synthetic route and method of Example 8, trifluoroisopropanol in step 1 was replaced by 4-fluorobutanol to obtain the target compound 063 (45 mg). LC-MS (ESI-MS): 455[M+H]⁺.

### Biological assay

### Example 64: KCNQ2/3 potassium ion channel activation assay

### Steps

### 1. Cell culture

hKCNQ-CHO cells were cultured at 37°C in a humidified environment of 5% CO₂.

### 2. Solution

2.1. Extracellular fluid (mM): NaCl 145, KCl 4, CaCl₂ 2, MgCl₂ 1, Glucose 10, HEPES 10. The pH value of the extracellular fluid was adjusted to 7.4 with NaOH, and the osmotic pressure was adjusted to about 295 mOsm.

2.2. Intracellular fluid (mM (mmol/L)): KOH 31.25, KCl 120, EGTA 10, MgCh 1.75, CaCl₂ 5.374, HEPES 10, Na-ATP 4. The pH value of the intracellular fluid was adjusted to 7.4 with HCl, and the osmotic pressure was adjusted to about 285 mOsm.

2.3. The extracellular fluid should be prepared once a week, and the intracellular fluid should be stored at -20°C after preparation.

### 3. Preparation of working solutions

The model positive reference compound Retigabine (RTG) was dissolved with 100% DMSO and configured as a 10 mM working solution. The test compounds were dissolved with 100% DMSO and configured as a 10 mM working solution. The final concentration of DMSO in the test solution should be less than 0.3%.

### 4. Whole-cell voltage-clamp recording

Whole-cell patch clamp was performed at room temperature. The electrical signals recorded with the EPC 10 USB amplifier (HEKA Elektronik, Germany) were filtered by a 3 kHz low-pass filter, and finally recorded in PatchMaster 2 × 90.5 software (HEKA Elektronik, Germany). At this step, the quality control standard was that the cell high-resistance sealing was greater than 500 MOhms, and the detection current was greater than 0.4 nA.

The recording electrodes were pulled out and polished with a vertical puller (NARISHIGE PC-10, Japan) using a borosilicate glass capillary (GC150tF-10, Harvard Apparatus Co., UK). At this step, the quality control standard was that the electrode resistance was between 2 MΩ and 5 MΩ.

During the recording process of the whole-cell patch clamp, a continuous perfusion system (BT100-2J, LongerPump, China) was used to continuously perfuse the extracellular fluid. The perfusion system was installed on the stage of an upright microscope (FN-S2N, Nikon, Japan), and the perfusion head was manually positioned under the microscope.

The voltage command for detecting the amplitude of hKCNQ current was as follows: the cell stepped from the clamping potential of -80 mV to -30 mV, and held for 1500 ms; and then the clamping voltage was lowered to -120 mv and held for 500 ms; finally, the voltage returned to the clamping potential of -80 mV. The detection voltage command was repeated every 15,000 milliseconds, and the command was continuously executed during the compound test. The quality control standard for compound action stability was the current amplitude under 5 consecutive voltage commands, and the coefficient of variation of the current amplitude was < 5%. If the compound did not affect the hKCNQ current amplitude, continuous monitoring for 5 minutes was required.

### 5. Data analysis

Data analysis was performed using PatchMaster 2 × 90.5 software (HEKAElektronik, Germany) and Excel 2013 (Microsoft, USA) software. For each cell's patch clamp recording, the amplitude of the hKCNQ channel current (Itest) corresponding to the test compound concentration should be obtained. The enhanced channel current was obtained by subtracting the cell's original channel current (Icontrol) from Itest and dividing by Icontrol to obtain the current enhancement multiple. That is, the current enhancement multiple (**Enhancment%**) = (Itest - Icontrol) / Icontrol * 100%. The experimental results are shown in Table 1

**Table 1 Activation activity of compounds on KCNQ2/3 potassium ion channel**

| **Compound No.** | **Enhancment%** | | **No.** | **Enhancment%** | |
|---|---|---|---|---|---|
| | **0.05 µM** | **3 µM** | | **0.05 µM** | **3 µM** |
| 001 | B | B | 002 | C | B |
| 003 | C | B | 004 | C | A |
| 005 | C | A | 006 | - | A |
| 007 | - | A | 008 | B | A |
| 009 | - | A | 010 | A | A |
| 011 | A | A | 012 | - | A |
| 013 | - | A | 014 | C | B |
| 015 | - | A | 017 | C | B |
| 022 | - | B | 023 | - | A |
| 024 | - | B | 025 | - | A |
| 026 | - | A | 027 | C | A |
| 028 | C | B | 029 | - | B |
| 030 | - | B | 031 | - | A |
| 032 | B | A | 033 | - | B |
| 034 | - | A | 035 | B | A |
| 036 | - | A | 037 | - | A |
| 038 | - | A | 039 | - | A |
| 040 | - | A | 041 | - | A |
| 042 | - | A | 043 | - | A |
| 044 | - | A | 045 | - | A |
| 046 | - | A | 047 | - | A |
| 048 | - | A | 049 | - | A |
| 050 | - | A | 051 | - | A |
| 052 | - | A | 053 | - | A |
| 054 | - | A | 055 | - | A |
| 056 | - | A | 057 | - | A |
| 058 | - | A | 059 | - | A |
| 060 | - | A | 061 | - | A |
| 062 | - | A | 063 | - | A |
| Compound a | - | 41.39 | Retigabine | 25.41^{a} | |

In the table: "0.05 µM" and "3 µM" indicate the test concentration of the test compounds. A > 100%, 50% < B < 100%, C < 50%. "-" indicates that no corresponding test is performed.

Where compound a is a reference compound, and its structure is 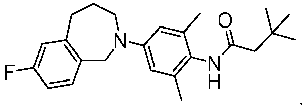 ^{a} represents the activation activity of Retigabine on KCNQ2/3 potassium ion channel at a concentration of 10 µM.

The result of Table 1 indicates that the activation activity of compound a to KCNQ2/3 potassium ion channel at the concentration of 3 µM and that of Retigabine at the concentration of 10 µM are lower than 50%, and the activation activity of the compounds of the present disclosure to KCNQ2/3 potassium ion channel at the concentration of 3 µM is greater than 50%.

### Example 65: half-maximal activation voltage left shift assay

### Steps

### 1. Cell culture

hKCNQ-CHO cells were cultured at 37°C in a humidified environment of 5% COz.

### 2. Solution

a) Extracellular fluid (mM): NaCl 145, KCl 4, CaCh 2, MgCh 1, Glucose 10, HEPES 10. The pH value of the extracellular fluid was adjusted to 7.4 with NaOH, and the osmotic pressure was adjusted to about 295 mOsm.
b) Intracellular fluid (mM): KOH 31.25, KCl 120, EGTA 10, MgClz 1.75, CaClz 5.374, HEPES 10, Na-ATP 4. The pH value of the intracellular fluid was adjusted to 7.4 with HCl, and the osmotic pressure was adjusted to about 285 mOsm.
c) The extracellular fluid should be prepared once a week, and the intracellular fluid should be aliquoted and stored at -20°C after preparation.

### 3. Preparation of working solutions

The model positive reference compound Retigabine (RTG) was dissolved with 100% DMSO and configured as a 10 mM working solution. The test compounds were dissolved with 100% DMSO and configured as a 10 mM working solution. The final concentration of DMSO in the test solution should be less than 0.3%.

### 4. Whole-cell voltage-clamp recording

Whole-cell patch clamp was performed at room temperature. The electrical signals recorded with the EPC 10 USB amplifier (HEKA Elektronik, Germany) were filtered by a 3 kHz low-pass filter, and finally recorded in PatchMaster 2 × 90.5 software (HEKA Elektronik, Germany). At this step, the quality control standard was that the cell high-resistance sealing was greater than 500 MOhms, and the detection current was greater than 0.4 nA.

The recording electrodes were pulled out and polished with a vertical puller (NARISHIGE PC-10, Japan) using a borosilicate glass capillary (GC150tF-10, Harvard Apparatus Co., UK). At this step, the quality control standard was that the electrode resistance was between 2 MΩ and 5 MΩ.

During the recording process of the whole-cell patch clamp, a continuous perfusion system (BT100-2J, LongerPump, China) was used to continuously perfuse the extracellular fluid. The perfusion system was installed on the stage of an upright microscope (FN-S2N, Nikon, Japan), and the perfusion head was manually positioned under the microscope.

The voltage command for detecting the amplitude of hKCNQ current was as follows: the cell was clamped from a potential of -80 mV, stepped to -120 mV, and then clamped continuously at increasing voltages of 10 mV per sweep until +60 mV, held for 1500 ms; and then the clamping voltage was lowered to -120 mv and held for 500 ms; finally, the voltage returned to the clamping potential of -80 mV. The detection voltage command was repeated every 15,000 milliseconds, and the command was continuously executed during the compound test. The half-maximal voltage of the cell's hKCNQ was obtained by nonlinear fitting of the current amplitude at different clamping potentials (-120 mV to +60 mV).

### 5. Data analysis

Data analysis was performed using PatchMaster 2 × 90.5 software (HEKAElektronik, Germany) and Excel 2013 (Microsoft, USA) software. For each cell's patch clamp recording, the half-maximal voltage (V1/2-test) of hKCNQ corresponding to the test compound concentration should be obtained. The left-shifted half-maximal voltage value ΔV1/2 (**V_{1/2} Shift (mV))** was obtained by subtracting the half-maximal voltage of hKCNQ before compound action (V1/2-control) from V1/2-test.

The experimental results are shown in Table 2:

**Table 2 Left shift effects of compounds on KCNQ2/3 half-maximal activation voltage**

| **Compound No.** | **V_{1/2} Shift (mV)** | | **No.** | **V_{1/2} Shift (mV)** | |
|---|---|---|---|---|---|
| | **50 nM** | **300 nM** | | **50 nM** | **300 nM** |
| 003 | -7.00 | -36.06 | 004 | -11.76 | -23.56 |
| 008 | -23.38 | -47.60 | 011 | -2.92 | -21.98 |
| 032 | -17.59 | - | 033 | -44.25 | - |
| 035 | -16.46 | - | | | |
| Xen-1101 | -5.48 | -13.33 | | | |

In the table: **"50 nM", "300 nM"** are the test concentrations of the test compounds. "-" indicates that no corresponding test is performed.

Compound Xen-1101 is a reference compound, its structure is: 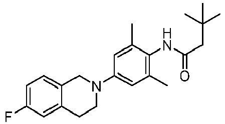 (WO2008024398A2)

The test result in Table 2 indicates that the compounds designed in the present disclosure have a significant effect in inducing a left shift in the half-maximal activation voltage.

### Example 66: blood-brain barrier permeability assay in mice

### Experimental procedure

### 1. Drug preparation

### Administration dosage of 5 mg/kg

The drug was prepared in proportion (solvent: 5% DMSO + 5% Solutol + 90% normal saline) to make a solution with a concentration of 0.5 mg/mL, and the mice were subjected to intragastric administration at 0.1 mL/10 g, with 3 mice per group in parallel.

### 2. Brain tissue collection at fixed points

After the mice were administered, the mice were anesthetized and perfused with normal saline through the heart, dissected to collect the brain tissue. The brain tissue was washed with normal saline, dried, weighed, and transferred to a 2 mL EP tube, added with normal saline according to brain tissue: normal saline = 1: 1 (g/mL). 3 steel balls were put in each tube on a tissue grinder, and the tissue mixture was homogenized at 60 HZ for 60 s, centrifuged at 15000 rpm at 4°C for 10 minutes, and the supernatant was taken, which was the brain tissue homogenate.

### 3. Sample processing

### 3.1. Preparation and processing of standard curve and quality control samples

The drug stock solution (0.5 mg/mL) was taken and diluted with acetonitrile to form standard curve working solutions with concentrations of 50, 100, 200, 500, 1000, 2000, 5000, 10000, and 20000 ng/mL, and quality control working solutions with concentrations of 100, 2000, and 10000 ng/mL. 47.5 µL of blank matrix was taken respectively, and 2.5 µL of standard curve working solution and 2.5 µL of quality control working solution were added thereto to prepare standard curve samples with concentrations of 2.5, 5, 10, 25, 50, 100, 250, 500, and 1000 ng/mL and quality control samples with concentrations of 5, 100, and 500 ng/mL. 200 µL of acetonitrile (containing the internal standard of 5 ng/mL of Loratadine) was added thereto respectively, subjected to vortex oscillation for 3 minutes, centrifuged at 15,000 rpm and 4°C for 10 minutes, and the supernatant was taken and analyzed by LC-MS/MS to obtain a standard curve.

### 3.2 Sample preparation and processing

50 µL of brain tissue homogenate sample was taken, added with 200 µL of acetonitrile (containing the internal standard of 5 ng/mL of Loratadine), subjected to vortex oscillation for 3 minutes, centrifuged at 15,000 rpm and 4°C for 10 minutes, and the supernatant was taken for LC-MS/MS analysis. The standard curve was combined with the analysis to obtain the corresponding concentrations in the brain.

The experimental results are shown in Table 3:

**Table 3 Concentrations of compounds in the brain after intragastric administration**

| Compound No. | Concentrations in the brain (ng/g) | Example No. | Concentrations in the brain (ng/g) |
|---|---|---|---|
| 003 | 127.5 | 008 | 166.1 |
| 035 | 257 | Xen-1101 | 90.9 |

The test results in Table 3 indicate that the concentrations of the compounds in the brain of the mice increase to a certain extent after intragastric administration.

### Example 67: MES mouse epilepsy model

### Experimental steps:

1. The mice (10 mice per group) were transferred to the operating room to adapt to the environment 30 minutes before the experiment.
2. The mice were administered the test compounds or positive drug (Valproate) at a specific time point. 30 minutes after the administration of Valproate or 30 minutes after the administration of the test compounds, electrical stimulation was given with ear electrodes. The stimulation parameters were a square wave with a total duration of 0.8 seconds, a frequency of 50 Hz, and an amplitude of 50 mA with an interval of 10 msec.
3. The number and duration of hind limb tonic extension of the animal within 1 minute after the completion of the electrical stimulation was recorded, and the possible death and time of death were also recorded.
4. After administration, the possible side effects induced by the drug were observed and recorded, and the side effects are divided into four levels:
   - None: Normal
   - Mild sedation
   - Moderate sedation
   - Severe sedation

The experimental results are shown in Table 4:

**Table 4 Protective effect of compounds on MES-induced epilepsy in mice (mpk: mg/kg)**

| Compound No. | Dosage | Protection rate for tonic extension |
|---|---|---|
| Example 003 | 5 mpk | 100% |
| Example 008 | 5 mpk | 90% |
| Example 011 | 5 mpk | 90% |
| Example 032 | 5 mpk | 100% |
| Example 033 | 5 mpk | 100% |
| Example 035 | 5 mpk | 100% |

The test results in Table 4 indicate that in MES-induced epilepsy in mice, the compounds can significantly inhibit the occurrence of hind limb tonic extension in mice.

## Claims

1. A compound, wherein the compound has a structure shown in general formula I: 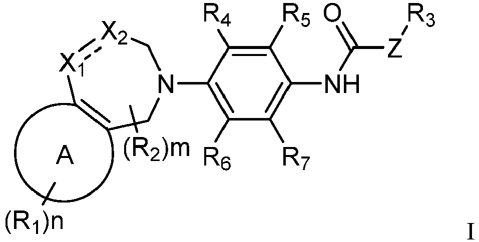
or a stereoisomer thereof, or a pharmaceutically acceptable salt thereof;
wherein
ring A is a benzene ring or a 5- to 8-membered heterocycle containing 1 to 2 heteroatoms selected from N, O, or S;
R₁ is selected from hydrogen, halogen, C₁-C₈ alkyl, C₁-C₈ alkoxy, C₁-C₈ alkenyl, C₁-C₈ alkenoxy, spiroalkyl, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₁-C₈ alkenoxy, halo C₃-C₈ cycloalkyl, halo C₃-C₈ cycloalkoxy, halo C₂-C₈ heterocycloalkyl, halo C₂-C₈ heterocycloalkoxy, and the alkyl, alkoxy, alkenyl, alkenoxy, cycloalkyl, cycloalkoxy, heterocycloalkyl, heterocycloalkoxy, alkylthio, alkanoyl, alkylsulfonyl, aminosulfonyl are unsubstituted or substituted by Rs;
each R₂ is optionally selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
or two R₂ groups are attached to the same atom, and the two R₂ groups are different or the same, and the two R₂ groups, together with the atom to which they are attached, form a 3- to 6-membered ring or a 3- to 6-membered heterocycle;
R₄, R₅, R₆, R₇, Rs are each independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy, spiroalkyl;
n is selected from 0, 1, 2;
m is selected from 0, 1, 2, 3, 4, 5;
X₁ and X₂ are each independently selected from -CRaRb, -NRa, -O-, -C(O)-, -S-, -S(O)-, -S(O)z-;
Ra and Rb are each independently selected from hydrogen, halogen, hydroxyl, amino, C₁-C₈ alkyl, C₁-C₈ alkoxy, C₁-C₈ alkylamino, C₃-C₈ cycloalkyl, halo C₁-C₈ alkyl, halo C₁-C₈ alkoxy, halo C₃-C₈ cycloalkyl;
- - - indicates the presence or absence of a chemical bond;
Z is selected from O or (CH₂)ₚ, and p is an integer of 1 to 6;
R₃ is C₁-C₈ alkyl, C₃-C₈ cycloalkyl, C₃-C₈ cycloalkenyl, C₂-C₈ alkenyl, or C₂-C₈ alkynyl, wherein the C₁-C₈ alkyl, C₃-C₈ cycloalkyl, C₃-C₈ cycloalkenyl, C₂-C₈ alkenyl, or C₂-C₈ alkynyl is unsubstituted or substituted by one or more than one substituent selected from halogen, nitro, cyano, amino, or hydroxyl.

2. The compound according to claim 1, wherein the compound has a structure shown in general formula I: 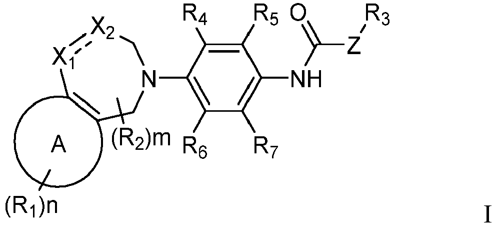
or a stereoisomer thereof, or a pharmaceutically acceptable salt thereof;
wherein
ring A is a benzene ring or a 5- to 8-membered heterocycle containing 1 to 2 heteroatoms selected from N, O, or S;
each R₁ is independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
each R₂ is independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
R₄, R₅, R₆, R₇ are each independently selected from hydrogen, halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy;
n is selected from 0, 1, 2;
m is selected from 0, 1, 2, 3, 4, 5;
X₁ and X₂ are each independently selected from -CRaRb, -NRa, -O-, -C(O)-, -S-, -S(O)-, -S(O)₂-;
Ra and Rb are each independently selected from hydrogen, halogen, hydroxyl, amino, C₁-C₈ alkyl, C₁-C₈ alkoxy, C₁-C₈ alkylamino, C₃-C₈ cycloalkyl, halo C₁-C₈ alkyl, halo C₁-C₈ alkoxy, halo C₃-C₈ cycloalkyl;
- - - indicates the presence or absence of a chemical bond;
Z is selected from O or (CH₂)ₚ, and p is an integer of 1 to 6;
R₃ is C₁-C₈ alkyl, C₃-C₈ cycloalkyl, C₃-C₈ cycloalkenyl, C₂-C₈ alkenyl, or C₂-C₈ alkynyl, wherein the C₁-C₈ alkyl, C₃-C₈ cycloalkyl, C₃-C₈ cycloalkenyl, C₂-C₈ alkenyl, or C₂-C₈ alkynyl is unsubstituted or substituted by one or more than one substituent selected from halogen, nitro, cyano, amino, or hydroxyl;
when x₁--x₂ is represented as x₁=x₂, X₁, X₂ are each independently selected from - CRa-, -N-;
when x₁--x₂ is represented as x₁-x₂, and when none of R₄, R₅, R₆, R₇ is selected from chlorine, and when X₁ is CH or O, R₂ is H, and when ring A is a benzene ring, R₁ is selected from C₁-C₈ alkoxy, C₃-C₈ cycloalkoxy, C₂-C₈ heterocycloalkoxy, C₁-C₈ alkylthio, C₁-C₈ alkanoyl, C₁-C₈ alkylsulfonyl, aminosulfonyl, halo C₁-C₈ alkoxy, halo C₃-C₈ cycloalkoxy, halo C₂-C₈ heterocycloalkoxy;
when x₁--x₂ is represented as x₁-x₂, and when none of R₄, R₅, R₆, R₇ is selected from chlorine, and when ring Ais a thiophene ring, m is selected from 2, 3, 4, 5, and R₂ is not selected from hydrogen.

3. The compound according to claim 2, wherein the compound has a structure shown in formulas IIa to IIc: 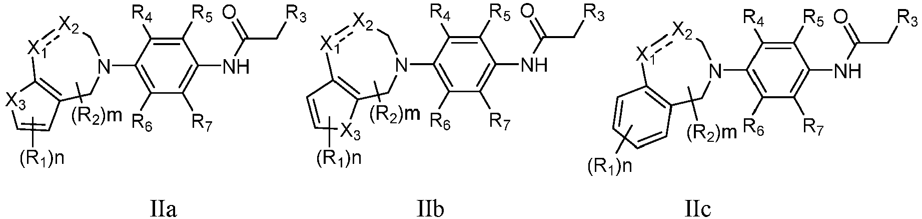
or a stereoisomer thereof, or a pharmaceutically acceptable salt thereof;
the bond between X₁ and X₂ is a double bond or a single bond;
X₃ is selected from N, O, S.

4. The compound according to claim 3, wherein the compound has a structure shown in formula III: 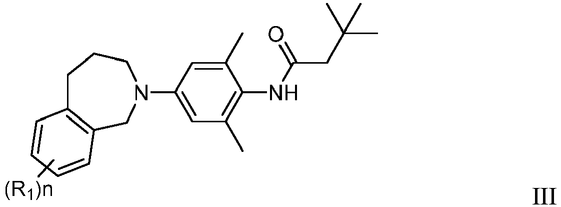
or a stereoisomer thereof, or a pharmaceutically acceptable salt thereof;
R₁ is selected from C₁₋₈ alkoxy, C₃₋₈ cycloalkoxy, C₂-C₈ heterocycloalkoxy, C₁-C₈ alkylthio, C₁-C₈ alkylsulfonyl, halo C₁₋₈ alkoxy, halo C₃₋₈ cycloalkoxy;
n is selected from 1.

5. The compound according to claim 4, wherein the compound has a structure shown in formula IV: 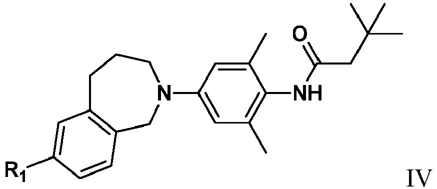
or a stereoisomer thereof, or a pharmaceutically acceptable salt thereof;
R₁ is selected from C₁₋₆ alkoxy, C₃₋₆ cycloalkoxy, C₂-C₆ heterocycloalkoxy, C₁-C₃ alkylthio, C₁-C₃ alkylsulfonyl, halo C₁₋₆ alkoxy, halo C₃₋₆ cycloalkoxy.

6. The compound according to claim 1, wherein the compound has a structure shown in formula IIc: 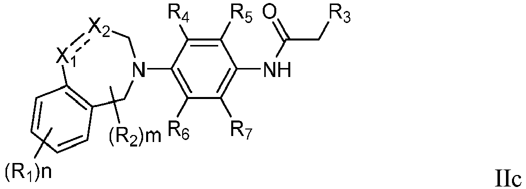
or a stereoisomer thereof, or a pharmaceutically acceptable salt thereof;
the bond between X₁ and X₂ is a double bond or a single bond;
n is selected from 2;
R₁ is selected from halogen, C₁-C₈ alkyl, C₁-C₈ alkoxy, C₃-C₈ cycloalkyl, C₃-C₈ cycloalkoxy, C₂-C₈ heterocycloalkyl, C₂-C₈ heterocycloalkoxy, C₁-C₈ alkylthio, C₁-C₈ alkanoyl, C₁-C₈ alkylsulfonyl, aminosulfonyl, halo C₁-C₈ alkyl, halo C₁-C₈ alkoxy, halo C₃-C₈ cycloalkyl, halo C₃-C₈ cycloalkoxy, halo C₂-C₈ heterocycloalkyl, halo C₂-C₈ heterocycloalkoxy.

7. The compound according to claim 6, wherein the compound has a structure shown in formula III: 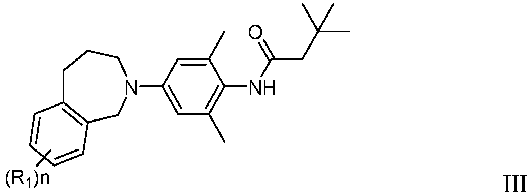
or a stereoisomer thereof, or a pharmaceutically acceptable salt thereof;
R₁ is selected from halogen, C₁₋₈ alkoxy, C₃₋₈ cycloalkoxy, C₂-C₈ heterocycloalkoxy, C₁-C₈ alkylthio, C₁-C₈ alkylsulfonyl, halo C₁₋₈ alkoxy, halo C₃₋₈ cycloalkoxy;
n is selected from 2.

8. The compound according to claim 7, wherein the compound has a structure shown in formula V: 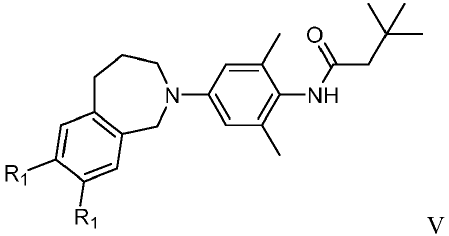
or a stereoisomer thereof, or a pharmaceutically acceptable salt thereof;
R₁ is selected from halogen, C₁₋₆ alkoxy, C₃₋₆ cycloalkoxy, C₂-C₆ heterocycloalkoxy, C₁-C₃ alkylthio, C₁-C₃ alkylsulfonyl, halo C₁₋₆ alkoxy, halo C₃₋₆ cycloalkoxy.

9. The compound according to claim 3, wherein in formulas IIa to IIc:
X₁, X₂ are CH₂ with a single bond between them; R₄ and R₆ are H; R₅ and R₇ are methyl;
m is selected from 2, 3, 4, 5;
R₂ is not selected from hydrogen.

10. The compound according to claim 3, wherein in formulas IIa to IIc:
R₆ is Cl; at least two of R₄, R₅, R₇ are not selected from hydrogen.

11. The compound according to claim 1, wherein the compound has a structure shown in formula Va: 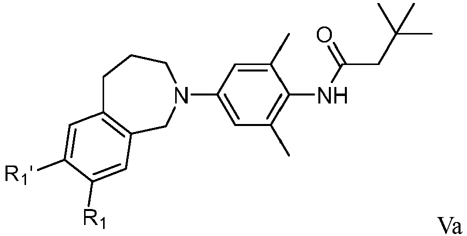
R₁' is H or F;
R₁ is

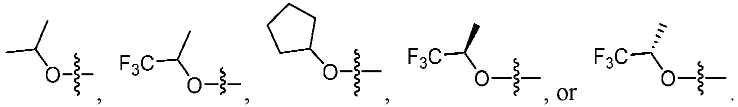

12. The compound according to claim 1, wherein the compound is selected from the following compounds:
| No. | Compound structure | No. | Compound structure |
|---|---|---|---|
| 001 | 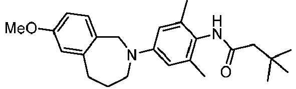 | 002 | 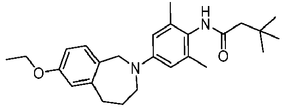 |
| 003 | 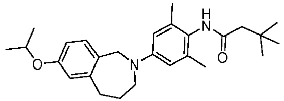 | 004 | 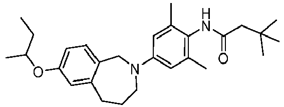 |
| 005 | 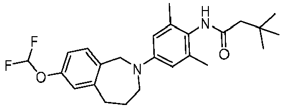 | 006 | 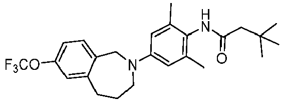 |
| 007 | 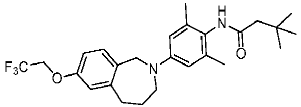 | 008 | 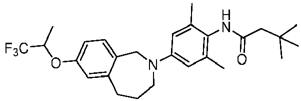 |
| 009 | 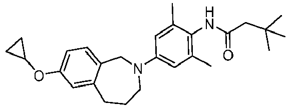 | 010 | 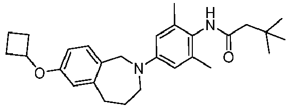 |
| 011 | 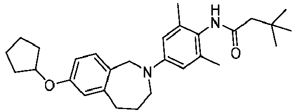 | 012 | 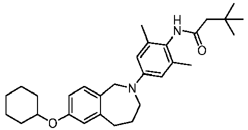 |
| 013 | 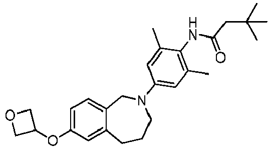 | 014 | 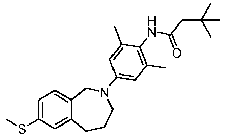 |
| 015 | 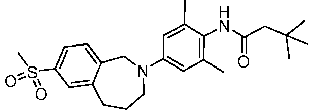 | 016 | 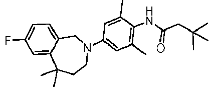 |
| 017 | 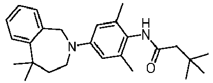 | 018 | 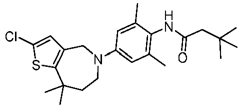 |
| 019 | 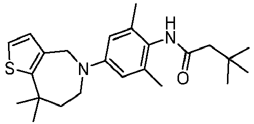 | 020 | 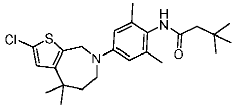 |
| 021 | 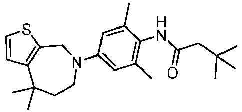 | 022 | 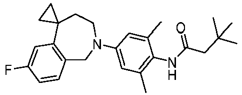 |
| 023 | 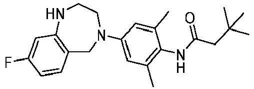 | 024 | 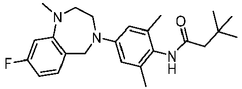 |
| 025 | 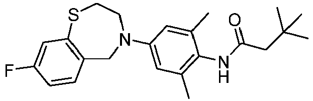 | 026 | 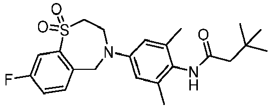 |
| 027 | 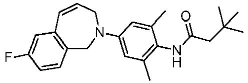 | 028 | 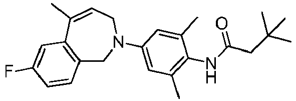 |
| 031 | 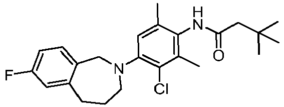 | 032 | 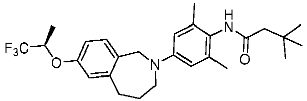 |
| 033 | 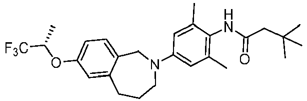 | 034 | 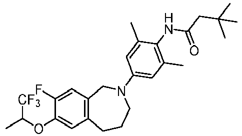 |
| 035 | 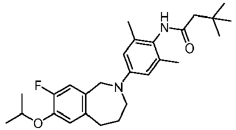 | 036 | 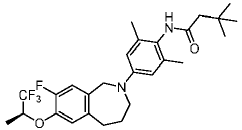 |
| 037 | 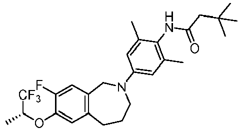 | 038 | 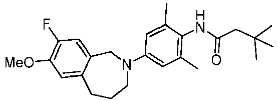 |
| 039 | 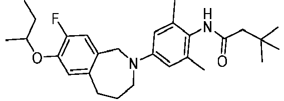 | 040 | 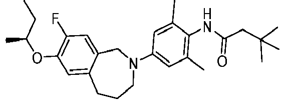 |
| 041 | 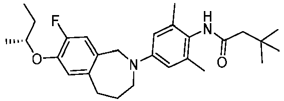 | 042 | 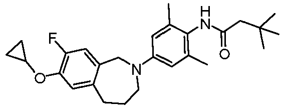 |
| 043 | 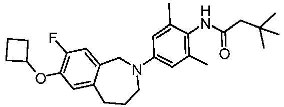 | 044 | 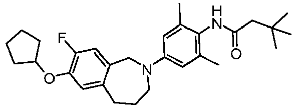 |
| 045 | 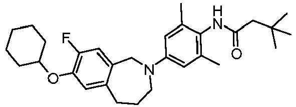 | 046 | 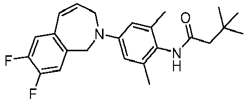 |
| 047 | 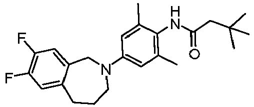 | 048 | 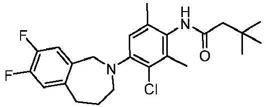 |
| 049 | 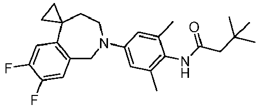 | 050 | 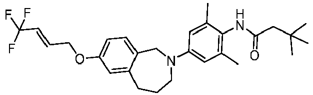 |
| 051 | 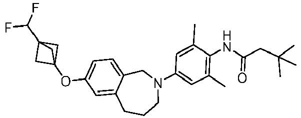 | 052 | 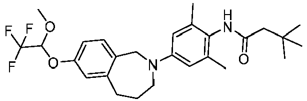 |
| 053 | 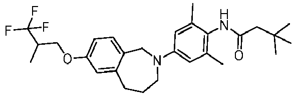 | 054 | 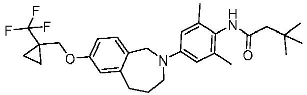 |
| 055 | 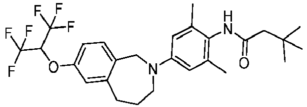 | 056 | 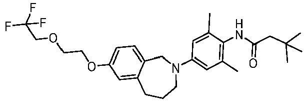 |
| 057 | 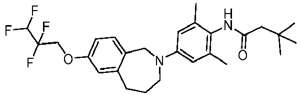 | 058 | 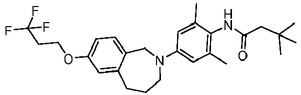 |
| 059 | 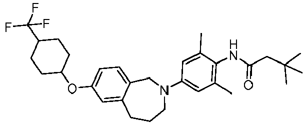 | 060 | 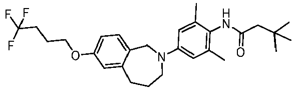 |
| 061 | 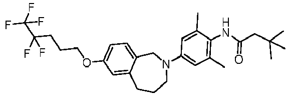 | 062 | 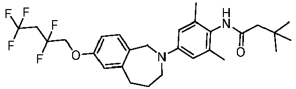 |
| 063 | 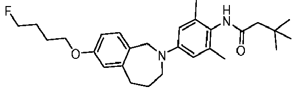 | | |
or a stereoisomer thereof, or a pharmaceutically acceptable salt thereof.

13. A pharmaceutical composition comprising one or more than one compound according to any of claims 1 to 12.

14. A pharmaceutical formulation comprising one or more than one compound according to any of claims 1 to 12.

15. A use of the compound or the pharmaceutically acceptable salt thereof according to any of claims 1 to 12 in the manufacture of a medicament for treating diseases benefiting from the activation of potassium ion channels, especially for treating central nervous system diseases.

16. The use according to claim 15, wherein the disease is selected from epilepsy, inflammatory pain, neuropathic pain, migraine, neurodegenerative diseases, anxiety disorders, depression, stroke, complications caused by cocaine abuse, nicotine withdrawal syndrome, alcohol withdrawal syndrome, or tinnitus.
